# EUROPEAN PATENT APPLICATION

(11) **EP 4 356 856 A1**
(43) Date of publication of application: **24.04.2024**
(21) Application number: 23204040.2
(22) Date of filing: 17.10.2023
(51) Int. Cl.: A61B 18/14, A61M 25/00, A61M 39/10, A61M 39/12

(54) **CATHETER INSERTION TOOL**

(30) Priority: 18.10.2022 US 202263417162 P; 29.12.2022 US 202263436022 P; 06.10.2023 US 202318482815
(71) Applicant: BIOSENSE WEBSTER (ISRAEL) LTD., 2066717 Yokneam (IL)
(72) Inventor: BEECKLER, Christopher T., Irvine, 92618 (US); OKARSKI, Kevin M., Irvine, 92618 (US); KEYES, Joseph T., Irvine, 92618 (US); HERERA, Kevin J., Irvine, 92618 (US); KATO, Robert B., Irvine, 92618 (US)
(74) Representative: Carpmaels & Ransford LLP

(57) **Abstract**

A delivery sheath to catheter coupler includes a cylindrical shaft and a coupling member. The cylindrical shaft extends along an axis and is configured for longitudinal alignment with an insertion port of a catheter delivery sheath. The cylindrical shaft includes a proximal end, a distal end, and a lumen extending from the proximal end to the distal end along the axis. The lumen is sized to receive an end effector of a catheter. The coupling member is attached to the cylindrical shaft and is configured for selective securement to the catheter delivery sheath. The coupling member is configured to align the axis of the lumen with a longitudinal axis of the insertion port when the coupling member is selectively secured to the catheter delivery sheath.

## Description

### CROSS-REFERENCE TO RELATED APPLICATION(S)

The present application claims priority to and the benefit of U.S. Provisional Patent Application No. 63/417,162, filed October 18, 2022, and U.S. Provisional Patent Application No. 63/436,022, filed December 29, 2022, the entire content of both of which is incorporated herein by reference.

### BACKGROUND

Cardiac arrhythmias, such as atrial fibrillation, occur when regions of cardiac tissue abnormally conduct electric signals. Procedures for treating arrhythmia include surgically disrupting the conducting pathway for such signals. By selectively applying electrical energy to cardiac tissue, it may be possible to cease or modify the propagation of unwanted electrical signals from one portion of the heart to another. Some such ablation treatments may include radiofrequency (RF) ablation with alternating current (AC) electrical energy; and/or irreversible electroporation (IRE) via pulsed field direct current (DC) electrical energy. The ablation process may provide a barrier to unwanted electrical pathways by creating electrically insulative lesions or scar tissue that effectively block communication of aberrant electrical signals across the ablated tissue.

In some procedures, a catheter with one or more electrodes may be used to provide ablation within a patient. The catheter may be inserted into a major vein or artery (e.g., the femoral artery) and then advanced to position the electrodes within the heart or in a structure adjacent to the heart (e.g., the pulmonary vein). The one or more electrodes may be placed in contact with cardiac tissue or other vascular tissue and then activated with electrical energy to thereby ablate the contacted tissue (e.g., via RF energy, IRE, etc.). In some cases, the electrodes may be bipolar. In some other cases, a monopolar electrode may be used in conjunction with a ground pad or other reference electrode that is in contact with the patient. Irrigation may be used to draw heat from components of an ablation catheter; and to prevent the formation of blood clots near the tissue treatment site.

Examples of ablation catheters are described in U.S. Pub. No. 2013/0030426, entitled "Integrated Ablation System using Catheter with Multiple Irrigation Lumens," published January 31, 2013, the disclosure of which is incorporated by reference herein, in its entirety; U.S. Pub. No. 2017/0312022, entitled "Irrigated Balloon Catheter with Flexible Circuit Electrode Assembly," published November 2, 2017, the disclosure of which is incorporated by reference herein, in its entirety; U.S. Pub. No. 2018/0071017, entitled "Ablation Catheter with a Flexible Printed Circuit Board," published March 15, 2018, the disclosure of which is incorporated by reference herein, in its entirety; U.S. Pub. No. 2018/0056038, entitled "Catheter with Bipole Electrode Spacer and Related Methods," published March 1, 2018, the disclosure of which is incorporated by reference herein, in its entirety; U.S. Pat. No. 10,130,422, entitled "Catheter with Soft Distal Tip for Mapping and Ablating Tubular Region," issued November 20, 2018, the disclosure of which is incorporated by reference herein, in its entirety; U.S. Pat. No. 8,956,353, entitled "Electrode Irrigation Using Micro-Jets," issued February 17, 2015, the disclosure of which is incorporated by reference herein, in its entirety; and U.S. Pat. No. 9,801,585, entitled "Electrocardiogram Noise Reduction," issued October 31, 2017, the disclosure of which is incorporated by reference herein, in its entirety.

Some catheter ablation procedures may be performed after using electrophysiology (EP) mapping to identify tissue regions that should be targeted for ablation. Such EP mapping may include the use of sensing electrodes on a catheter (e.g., the same catheter that is used to perform the ablation or a dedicated mapping catheter). Such sensing electrodes may monitor electrical signals emanating from conductive endocardial tissues to pinpoint the location of aberrant conductive tissue sites that are responsible for the arrhythmia. Examples of an EP mapping system are described in U.S. Pat. No. 5,738,096, entitled "Cardiac Electromechanics," issued April 14, 1998, the disclosure of which is incorporated by reference herein, in its entirety. Examples of EP mapping catheters are described in U.S. Pat. No. 9,907,480, entitled "Catheter Spine Assembly with Closely-Spaced Bipole Microelectrodes," issued March 6, 2018, the disclosure of which is incorporated by reference herein, in its entirety; U.S. Pat. No. 10,130,422, entitled "Catheter with Soft Distal Tip for Mapping and Ablating Tubular Region," issued November 20, 2018, the disclosure of which is incorporated by reference herein, in its entirety; and U.S. Pub. No. 2018/0056038, entitled "Catheter with Bipole Electrode Spacer and Related Methods," published March 1, 2018, the disclosure of which is incorporated by reference herein, in its entirety.

Some catheter ablation procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO 3^{®} system by Biosense Webster, Inc. of Irvine, California. Examples of catheters that are configured for use with an IGS system are disclosed in U.S. Pat. No. 9,480,416, entitled "Signal Transmission Using Catheter Braid Wires," issued November 1, 2016, the disclosure of which is incorporated by reference herein, in its entirety; and various other references that are cited herein.

While several catheter systems and methods have been made and used, it is believed that no one prior to the inventors has made or used the invention described, illustrated and claimed herein.

### SUMMARY OF THE DISCLOSURE

In some embodiments, a delivery sheath to catheter coupler comprises a cylindrical shaft and a coupling member. The cylindrical shaft extends along an axis and is configured for longitudinal alignment with an insertion port of a catheter delivery sheath. The cylindrical shaft includes a proximal end, a distal end, and a lumen extending from the proximal end to the distal end along the axis and the lumen is sized to receive an end effector of a catheter. The coupling member is attached to the cylindrical shaft and configured for selective securement to the catheter delivery sheath. The coupling member is further configured to align the axis of the lumen with a longitudinal axis of the insertion port when the coupling member is selectively secured to the catheter delivery sheath.

In some embodiments, the coupling member is fixedly attached to the cylindrical shaft.

In some embodiments, the coupling member is attached to the cylindrical shaft while also being movable relative to the cylindrical shaft.

In some embodiments, the coupling member is configured to translate relative to the cylindrical shaft along the axis of the lumen.

In some embodiments, the cylindrical shaft is sized for insertion into the insertion port of the catheter delivery sheath.

In some embodiments, the coupler further includes an outwardly flared feature at one of the proximal or distal ends of the cylindrical shaft. The outwardly flared feature defines an angled surface leading into the lumen.

In some embodiments, the outwardly flared feature is disposed at the distal end of the cylindrical shaft and configured to prevent insertion of the cylindrical shaft into the insertion port of the catheter delivery sheath.

In some embodiments, the outwardly flared feature is disposed at the proximal end of the cylindrical shaft and configured to limit insertion of the cylindrical shaft into the insertion port of the catheter delivery sheath.

In some embodiments, the coupling member includes a vent opening for venting fluid from a space between the coupling member and the catheter delivery sheath when the coupling member is selectively secured to the catheter delivery sheath.

In some embodiments, the coupling member includes a cap.

In some embodiments, the coupling member is configured to engage the catheter delivery sheath in a threadable manner.

In some embodiments, the coupling member is configured to engage the catheter delivery sheath in a snap-fit manner.

In some embodiments, the coupling member is configured to engage the catheter delivery sheath in a bayonet-style manner.

In some embodiments, the coupler further comprises a catheter disposed in the lumen and the catheter is configured to fit within an anatomical structure.

In some embodiments, the coupler further comprises a catheter delivery sheath having an insertion port configured for longitudinal alignment with the cylindrical shaft. The insertion port defines a longitudinal axis and the coupling member is configured to align the axis of the lumen with the longitudinal axis of the insertion port when the coupling member is selectively secured to the catheter delivery sheath.

In some embodiments, a kit, comprises a catheter instrument and a coupler. The catheter instrument includes a catheter having a distal end, and an end effector at the distal end of the catheter. The catheter and the end effector are sized to fit in an anatomical structure and the end effector includes at least one electrode. The coupler includes a cylindrical shaft and a coupling member. The cylindrical shaft includes a proximal end, a distal end, and a lumen extending from the proximal end to the distal end along an axis, the lumen being sized to receive the end effector and the catheter. The coupling member is attached to the cylindrical shaft.

In some embodiments, the kit further comprises a catheter delivery sheath that includes an insertion port defining a longitudinal axis and the coupling member is configured for selective securement to the catheter delivery sheath and to align the axis of the lumen with the longitudinal axis of the insertion port when the coupling member is selectively secured to the catheter delivery sheath.

In some embodiments, the coupling member is configured to engage the catheter delivery sheath in at least one of a threadable manner, a snap-fit manner, or a bayonet-style manner.

In some embodiments, a kit comprises a catheter instrument and a coupler. The catheter instrument includes a catheter having a distal end, and an end effector at the distal end of the catheter. The catheter and the end effector are sized to fit in an anatomical structure and the end effector includes at least one electrode. The coupler includes a cylindrical shaft and means for coupling. The cylindrical shaft includes a proximal end, a distal end, and a lumen extending from the proximal end to the distal end along an axis, the lumen being sized to receive the end effector and the catheter. The means for coupling is attached to the cylindrical shaft.

In some embodiments, the means for coupling includes threading, a snap fit feature, a bayonet fit feature, or functional equivalents of threading, a snap fit feature, or a bayonet fit feature.

In some embodiments, a method comprises securing a coupler to a catheter delivery sheath and the coupler includes a cylindrical shaft and a coupling member. The cylindrical shaft includes a proximal end, a distal end, and a lumen extending from the proximal end to the distal end along an axis, the lumen being sized to receive an end effector and catheter of a catheter instrument. The coupling member is attached to the cylindrical shaft. The act of securing includes aligning the axis of the lumen with a longitudinal axis of an insertion port of the catheter delivery sheath.

In some embodiments, the act of securing includes providing at least one of a threadable engagement, a snap-fit engagement, or a bayonet-style engagement between the coupling member and the catheter delivery sheath.

In some embodiments, the lumen includes a frustoconical proximal portion.

In some embodiments, the frustoconical proximal portion extends distally from the proximal end.

In some embodiments, the frustoconical proximal portion tapering radially inwardly in a distal direction.

In some embodiments, the lumen including a frustoconical proximal portion.

In some embodiments, the frustoconical proximal portion extending distally from the proximal end.

In some embodiments, the frustoconical proximal portion tapering radially inwardly in a distal direction.

In some embodiments, a delivery sheath to catheter coupler comprises a cylindrical shaft and a cap. The cylindrical shaft extends along an axis and is configured for longitudinal alignment with an insertion port of a catheter delivery sheath. The cylindrical shaft includes a proximal end, a distal end, and a lumen extending from the proximal end to the distal end along the axis, the lumen being sized to receive an end effector of a catheter, the lumen including a frustoconical proximal portion. The cap is attached to the distal end of the cylindrical shaft and is configured for selective alignment with the catheter delivery sheath, and to align the axis of the lumen with a longitudinal axis of the insertion port when the coupling member is selectively aligned with the catheter delivery sheath.

In some embodiments, the cap is configured for selective securement to the catheter delivery sheath.

In some embodiments, the cap is fixedly attached to the distal end of the cylindrical shaft.

In some embodiments, the cap is attached to the distal end of the cylindrical shaft while also being movable relative to the cylindrical shaft.

In some embodiments, the cap is configured to translate relative to the cylindrical shaft along the axis of the lumen.

In some embodiments, the cap is configured to engage the catheter delivery sheath in at least one of a threadable manner, a snap-fit manner, an interference-fit manner, or a bayonet-style manner.

In some embodiments, the coupler further comprises a catheter disposed in the lumen, the catheter being configured to fit within an anatomical structure.

In some embodiments , the coupler further comprises a catheter delivery sheath having an insertion port configured for longitudinal alignment with the cylindrical shaft. The insertion port defines a longitudinal axis and the coupling member is configured to align the axis of the lumen with the longitudinal axis of the insertion port when the coupling member is aligned with the catheter delivery sheath.

### BRIEF DESCRIPTION OF THE DRAWINGS

The drawings and detailed description that follow are intended to be merely illustrative and are not intended to limit the scope of the invention as contemplated by the inventors.
FIG. 1 depicts a schematic view of a medical procedure in which a catheter of a catheter assembly is inserted in a patient;
FIG. 2 depicts a perspective view of the catheter assembly of FIG. 1, with an end effector in an expanded state;
FIG. 3 depicts a perspective view of an example of a delivery sheath that may be used with the catheter assembly of FIG. 1;
FIG. 4A depicts a perspective view of an example of an insert member disposed on a distal portion of a catheter of the catheter assembly of FIG. 1, with the insert member and catheter positioned for insertion in the proximal end of the delivery sheath of FIG. 3;
FIG. 4B depicts a perspective view of the insert member of FIG. 4A disposed on a distal portion of a catheter of the catheter assembly of FIG. 1, with the insert member being inserted into the proximal end of the delivery sheath of FIG. 3, and with the catheter not yet inserted into the proximal end of the delivery sheath;
FIG. 4C depicts a perspective view of the insert member of FIG. 4A disposed on a distal portion of a catheter of the catheter assembly of FIG. 1, with the insert member and the catheter both inserted into the proximal end of the delivery sheath of FIG. 3;
FIG. 5 depicts an exploded perspective view of another example of an insert member and another example of a handle assembly that may be used with the catheter assembly of FIG. 1 and the delivery sheath of FIG. 3, the insert member including a coupling member in the form of a threaded cap;
FIG. 6A depicts a perspective view of the insert member of FIG. 5 positioned for coupling with an insertion port of the handle assembly of FIG. 5;
FIG. 6B depicts a perspective view of the insert member of FIG. 5 advanced distally to position the cap of the insert member over the insertion port of the handle assembly of FIG. 5;
FIG. 6C depicts a perspective view of the insert member of FIG. 5 rotated to threadably engage threads of the cap with threads of the insertion port of the handle assembly of FIG. 5 to secure the insert member to the handle assembly of FIG. 5;
FIG. 6D depicts a perspective view of the insert member of FIG. 5 secured to the handle assembly of FIG. 5 and guiding insertion of the catheter assembly of FIG. 1 into the delivery sheath of FIG. 3;
FIG. 7 depicts an exploded perspective view of an example of an insert member assembly and an example of a sheath hub that may be used with the catheter assembly of FIG. 1 and the delivery sheath of FIG. 3, the insert member assembly including a coupling member in the form of a cap having at least one L-shaped bayonet slot;
FIG. 8A depicts a perspective view of the insert member assembly of FIG. 7 positioned for coupling with a sideport of the sheath hub of FIG. 7;
FIG. 8B depicts a perspective view of the insert member assembly of FIG. 7 advanced distally to position the cap of the insert member assembly over at least an insertion port of the sheath hub of FIG. 7;
FIG. 8C depicts a perspective view of the insert member assembly of FIG. 7 rotated to engage the L-shaped bayonet slot of the cap with the sideport of the sheath hub of FIG. 7 to secure the insert member assembly to the sheath hub;
FIG. 8D depicts a perspective view of the insert member assembly of FIG. 7 secured to the sheath hub of FIG. 7 and arresting insertion of the catheter assembly of FIG. 1 into the delivery sheath of FIG. 3;
FIG. 9 depicts an exploded perspective view of another example of an insert member assembly that may be used with the catheter assembly of FIG. 1 and the delivery sheath of FIG. 3, the insert member assembly including a coupling member in the form of a cap having at least one J-shaped bayonet slot;
FIG. 10A depicts a perspective view of the insert member assembly of FIG. 9 positioned for coupling with the sideport of the sheath hub of FIG. 7;
FIG. 10B depicts a perspective view of the insert member assembly of FIG. 9 advanced distally to position the cap of the insert member assembly over at least the insertion port of the sheath hub of FIG. 7;
FIG. 10C depicts a perspective view of the insert member assembly of FIG. 9 rotated to engage the J-shaped bayonet slot of the cap with the sideport of the sheath hub of FIG. 7 to secure the insert member assembly to the sheath hub;
FIG. 10D depicts a perspective view of the insert member assembly of FIG. 9 secured to the sheath hub of FIG. 7 and arresting insertion of the catheter assembly of FIG. 1 into the delivery sheath of FIG. 3;
FIG. 11 depicts a perspective view of another exemplary coupling member in the form of a cap having at least one slanted bayonet slot for use with the insert member assembly of FIG. 9;
FIG. 12 depicts a perspective view of another example of an insert member assembly that may be used with the catheter assembly of FIG. 1 and the delivery sheath of FIG. 3, the insert member assembly including a coupling member in the form of a cap having a cylindrical sidewall with at least one snap-fit slot;
FIG. 13A depicts a perspective view of the insert member assembly of FIG. 12 positioned for coupling with the sideport of the sheath hub of FIG. 7;
FIG. 13B depicts a perspective view of the insert member assembly of FIG. 12 advanced distally to engage the snap-fit slot of the cap with the sideport of the sheath hub of FIG. 7 to secure the insert member assembly to the sheath hub;
FIG. 13C depicts a perspective view of the insert member assembly of FIG. 12 secured to the sheath hub of FIG. 7 and arresting insertion of the catheter assembly of FIG. 1 into the delivery sheath of FIG. 3;
FIG. 14 depicts a perspective view of another example of an insert member assembly that may be used with the catheter assembly of FIG. 1 and the delivery sheath of FIG. 3, the insert member assembly including a coupling member in the form of a cap having a pair of sidewalls with respective snap-fit slots;
FIG. 15 depicts a perspective view of another example of an end effector that may be integrated into the catheter assembly of FIG. 1, the end effector having an expandable basket configuration, showing the end effector in an expanded state;
FIG. 16 depicts a cross-sectional side view of a hollow shaft of the delivery sheath of FIG. 3, showing the end effector of FIG. 15 positioned within the hollow shaft and in a non-expanded state;
FIG. 17 depicts an exploded perspective view of the handle assembly of FIG. 3 and another example of an insert member that may be used with the catheter assembly of FIG. 1 and the end effector of FIG. 15, the insert member including a coupling member in the form of a cap;
FIG. 18A depicts a perspective view of the insert member of FIG. 17 positioned for coupling with an insertion port of the handle assembly of FIG. 3;
FIG. 18B depicts a perspective view of the insert member of FIG. 17 advanced distally to position the cap of the insert member over the insertion port of the handle assembly of FIG. 3; and
FIG. 18C depicts a perspective view of the insert member of FIG. 17 secured to the handle assembly of FIG. 3 and guiding insertion of the catheter assembly of FIG. 1 into the delivery sheath of FIG. 3.

### DETAILED DESCRIPTION FOR MODES OF CARRYING OUT THE INVENTION

The following description of certain examples of the invention should not be used to limit the scope of the present invention. The drawings, which are not necessarily to scale, depict selected embodiments and are not intended to limit the scope of the invention. The detailed description illustrates by way of example, not by way of limitation, the principles of the invention. Other examples, features, aspects, embodiments, and advantages of the invention will become apparent to those skilled in the art from the following description, which is by way of illustration, one of the best modes contemplated for carrying out the invention. As will be realized, the invention is capable of other different or equivalent aspects, all without departing from the invention. Accordingly, the drawings and descriptions should be regarded as illustrative in nature and not restrictive.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%. In addition, as used herein, the terms "patient," "host," "user," and "subject" refer to any human or animal subject and are not intended to limit the systems or methods to human use, although use of the subject invention in a human patient represents a preferred embodiment.

### I. Overview of Example of a Catheter System

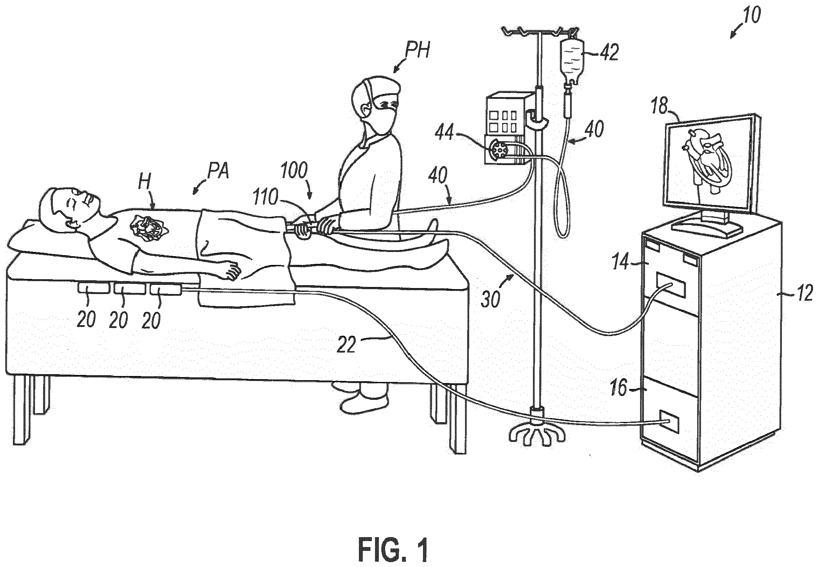

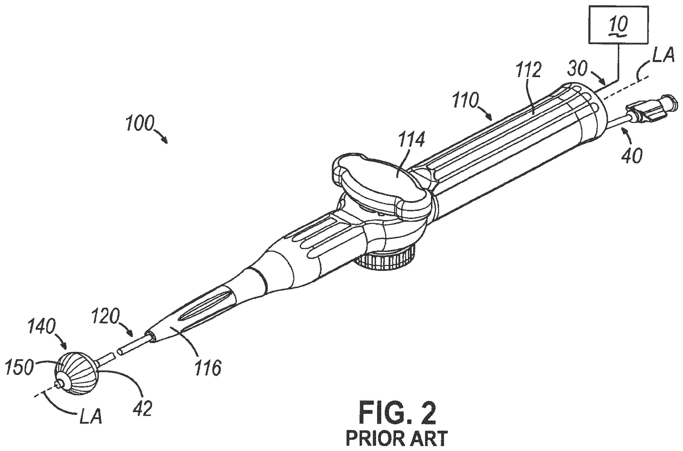

FIG. 1 shows an exemplary medical procedure and associated components of a cardiac catheter system that may be used to provide EP mapping or cardiac ablation as referred to above. In particular, FIG. 1 shows a physician (PH) grasping a handle assembly (110) of a catheter assembly (100), with an end effector (140) of a catheter (120) (shown in FIG. 2 but not shown in FIG. 1) of catheter assembly (100) disposed in a patient (PA) to map potentials in tissue and/or ablate tissue in or near the heart (H) of the patient (PA). As shown in FIG. 2, catheter assembly (100) includes handle assembly (110), catheter (120) extending distally from handle assembly (110), end effector (140) located at a distal end of catheter (120), and a deflection drive actuator (114) associated with handle assembly (110).

As will be described in greater detail below, end effector (140) includes various components configured to deliver electrical energy (e.g., RF energy and/or pulsed field DC energy, etc.) to targeted tissue sites, provide EP mapping functionality, track external forces imparted on end effector (140), track the location of end effector (140), and/or disperse irrigation fluid. Deflection drive actuator (114) is rotatable relative to a casing (112) of handle assembly (110) to thereby deflect end effector (140) and a distal portion of catheter (120) away from a central longitudinal axis (LA) defined by a proximal portion of catheter (120). Various suitable components that may be coupled with deflection drive actuator (114) and catheter (120) to provide such functionality will be apparent to those skilled in the art in view of the teachings herein.

As shown in FIG. 2, catheter (120) includes an elongate flexible shaft, with end effector (140) extending distally from the shaft. The proximal end of catheter (120) extends distally from a nozzle member (116) of handle assembly (110). In some versions, a heat shrink wrap (not shown) is provided about catheter (120), at the junction of the proximal end of catheter (120) and nozzle member (116). End effector (140) at the distal end of catheter (120) will be described in greater detail below. Catheter assembly (100) is coupled with a guidance and drive system (10) via a cable (30). Catheter assembly (100) is also coupled with a fluid source (42) via a fluid conduit (40). A set of field generators (20) are positioned underneath the patient (PA) and are coupled with guidance and drive system (10) via another cable (22). Field generators (20) are merely optional.

Guidance and drive system (10) of the present example include a console (12) and a display (18). Console (12) includes a first driver module (14) and a second driver module (16). First driver module (14) is coupled with catheter assembly (100) via cable (30). In some variations, first driver module (14) is operable to receive EP mapping signals obtained via the electrodes of end effector (140). Console (12) includes a processor (not shown) that processes such EP mapping signals and thereby provides EP mapping as is known in the art. In some other versions, end effector (140) includes other electrodes that are configured to provide EP mapping signals. In still other versions, end effector (140) does not provide EP mapping.

First driver module (14) of the present example is further operable to provide electrical power to the electrodes of end effector (140), as will be described in greater detail below, to thereby ablate tissue. In some other versions, end effector (140) includes other electrodes that are configured to provide ablation. In still other versions, end effector (140) does not provide ablation.

Second driver module (16) is coupled with field generators (20) via cable (22). Second driver module (16) is operable to activate field generators (20) to generate an alternating magnetic field around the heart (H) of the patient (PA). For instance, field generators (20) may include coils that generate alternating magnetic fields in a predetermined working volume that contains the heart (H).

First driver module (14) is also operable to receive position indicative signals from a navigation sensor assembly (not shown) in catheter (120) near end effector (140). In such versions, the processor of console (12) is also operable to process the position indicative signals from the navigation sensor assembly to thereby determine the position of end effector (140) within the patient (PA). In some versions, the navigation sensor assembly includes two or more coils that are operable to generate signals that are indicative of the position and orientation of end effector (140) within the patient (PA). The coils are configured to generate electrical signals in response to the presence of an alternating electromagnetic field generated by field generators (20). Other components and techniques that may be used to generate real-time position data associated with end effector (140) may include wireless triangulation, acoustic tracking, optical tracking, inertial tracking, and the like. While the navigation sensor assembly is shown as being disposed in the distal end of catheter (120), the navigation sensor assembly may instead be positioned in end effector (140). Alternatively, catheter (120) and end effector (140) may lack a navigation sensor assembly.

Display (18) is coupled with the processor of console (12) and is operable to render images of patient anatomy. Such images may be based on a set of preoperatively or intraoperatively obtained images (e.g., a CT or MRI scan, 3-D map, etc.). The views of patient anatomy provided through display (18) may also change dynamically based on signals from the navigation sensor assembly of end effector (140). For instance, as end effector (140) of catheter (120) moves within the patient (PA), the corresponding position data from the navigation sensor assembly may cause the processor of console (12) to update the patient anatomy views in display (18) in real time to depict the regions of patient anatomy around end effector (140) as end effector (140) moves within the patient (PA). Moreover, the processor of console (12) may drive display (18) to show locations of aberrant conductive tissue sites, as detected via electrophysiological (EP) mapping with end effector (140) or as otherwise detected (e.g., using a dedicated EP mapping catheter, etc.). By way of example only, the processor of console (12) may drive display (18) to superimpose the locations of aberrant conductive tissue sites on the images of the patient's anatomy, such as by superimposing an illuminated dot, a crosshair, or some other form of visual indication of aberrant conductive tissue sites.

The processor of console (12) may also drive display (18) to superimpose the current location of end effector (140) on the images of the patient's anatomy, such as by superimposing an illuminated dot, a crosshair, a graphical representation of end effector (140), or some other form of visual indication. Such a superimposed visual indication may also move within the images of the patient anatomy on display (18) in real time as the physician moves end effector (140) within the patient (PA), thereby providing real-time visual feedback to the operator about the position of end effector (140) within the patient (PA) as end effector (140) moves within the patient (PA). The images provided through display (18) may thus effectively provide a video tracking the position of end effector (140) within a patient (PA), without necessarily having any optical instrumentation (i.e., cameras) viewing end effector (140). In the same view, display (18) may simultaneously visually indicate the locations of aberrant conductive tissue sites detected through EP mapping. The physician (PH) may thus view display (18) to observe the real time positioning of end effector (140) in relation to the mapped aberrant conductive tissue sites and in relation to images of the adjacent anatomical structures in the patient (PA).

Fluid source (42) of the present example includes a bag containing saline or some other suitable irrigation fluid. Conduit (40) includes a flexible tube that is further coupled with a pump (44), which is operable to selectively drive fluid from fluid source (42) to catheter assembly (100). Such irrigation fluid may be expelled through openings (not shown) of end effector (140). Such irrigation may be provided in any suitable fashion as will be apparent to those skilled in the art in view of the teachings herein.

### II. Example of an End Effector

As mentioned above, end effector (140) includes various components configured to deliver electrical energy (e.g., RF energy and/or pulsed field DC energy, etc.) to targeted tissue sites, provide EP mapping functionality, track external forces imparted on end effector (140), track the location of end effector (140) within the patient (PA), and emit irrigation fluid. However, it should be understood that the version of end effector (140) is just one merely illustrative example. Various other kinds of end effectors may be used, including but not limited to end effectors having a spiral configuration, end effectors having a basket configuration, end effectors having a wire frame configuration, or end effectors having any other suitable configuration.

As shown in FIG. 2, end effector (140) of the present example includes an expandable balloon (142) and a set of electrode assemblies (150) that are angularly spaced apart from each other about balloon (142). Balloon (142) is operable to transition between a non-expanded state (not shown) and an expanded state (FIG. 2). Balloon (142) may be inflated with irrigation fluid (e.g., saline) from fluid source (42), with pump (44) providing pressure to the fluid, to transition from the non-expanded state to the expanded state. In the non-expanded state, balloon (142) may fit within a sheath, such as delivery sheath (200) described below or a sheath that is an integral part of catheter (120). In the expanded state, balloon (142) may be sized and configured to urge electrodes of electrode assemblies (150) into contact with tissue (e.g., the inner wall of a pulmonary vein or chamber of the heart (H)). In the present example, balloon (142) is formed of a flexible yet non-extensible material.

Each electrode assembly (150) of the present example includes a flexible substrate and an electrode, which may be formed as a flex circuit. The electrodes of the present example are operable to ablate tissue that is in contact with an electrode. The electrodes may be further constructed and operable in accordance with at least some of the teachings of U.S. Pub. No. 2017/0312022, entitled "Irrigated Balloon Catheter with Flexible Circuit Electrode Assembly," published November 2, 2017, the disclosure of which is incorporated by reference herein.

In some versions, the electrodes are configured to provide both electrical energy (e.g., RF or IRE, etc.) ablation functionality and EP mapping functionality. In some other versions, the electrodes are configured to provide only electrical energy ablation functionality without also providing EP mapping functionality. In still other versions, the electrodes are only configured to provide EP mapping functionality without also providing electrical energy ablation functionality. As yet another merely illustrative example, end effector (140) may include some electrodes that are dedicated to providing only electrical energy ablation functionality and other electrodes that are dedicated to providing only EP mapping functionality. Other suitable configurations and functionalities that may be associated with the electrodes will be apparent to those skilled in the art in view of the teachings herein.

In addition to the foregoing, end effector (140) and other aspects of catheter assembly (100) may be configured and operable in accordance with at least some of the teachings of U.S. Pub. No. 2017/0312022, entitled "Irrigated Balloon Catheter with Flexible Circuit Electrode Assembly," published November 2, 2017, the disclosure of which is incorporated by reference herein; and/or U.S. Pub. No. 2021/0322094, entitled "Pressure Relief Feature for Irrigated RF Balloon Catheter," published October 21, 2021, the disclosure of which is incorporated by reference herein. Alternatively, end effector (140) may have any other suitable components, features, and capabilities.

As indicated above, end effector (140) may have any other suitable type of expandable balloon configuration, any suitable type of expandable basket configuration, or any other suitable type of expandable configuration. In some versions, end effector (140) may be configured to define a spiral shape when in the expanded state. For example, end effector (140) may be configured and operable in accordance with one or more teachings of U.S. Pub. No. 2021/0085386, entitled "Catheter Instrument with Three Pull Wires," published March 25, 2021, the disclosure of which is incorporated by reference herein. In some other versions, end effector (140) may include a plurality of loop members comprising respective pairs of spines that are configured to splay out when in the expanded state. For example, end effector (140) may be configured and operable in accordance with one or more teachings of U.S. Pub. No. 2020/0345262, entitled "Mapping Grid with High Density Electrode Array," published November 5, 2021, the disclosure of which is incorporated by reference herein. Alternatively, end effector (140) may take any other suitable form.

### III. Example of Delivery Sheath

In some procedures, the physician (PH) may wish to introduce catheter (120) into the patient (PA) via a delivery sheath. In some such procedures, the delivery sheath may be inserted into the patient (PA) (e.g., via the leg or groin of the patient (PA)); and then be advanced along a vein or artery to reach a position in or near the heart (H). Once the delivery sheath is suitably positioned in the patient (PA), the physician (PH) may then advance end effector (140) and catheter (120) into the delivery sheath. End effector (140) may be in the non-expanded state during transit from the insertion site to the targeted region within the patient (PA). Once end effector (140) is suitably positioned near the targeted structure, the delivery sheath may be retracted relative to end effector (140) (or end effector (140) may be advanced relative to the delivery sheath). End effector (140) may then be expanded to the state shown in FIG. 2 by inflating balloon (142) to bring the electrodes into contact with the tissue of the targeted structure. In addition, or in the alternative, one or more resilient features of end effector (140) may urge end effector (140) from the non-expanded state (not shown) to the expanded state (FIG. 2). Once end effector (140) is in the expanded state, the physician (PH) may then operate catheter assembly (100) to provide EP mapping, ablation, or any other kind of operations in or near the heart (H) of the patient (PA). End effector (140) may then be collapsed to the non-expanded configuration by deflating balloon (142), and/or otherwise contracting end effector (140), for retraction through the delivery sheath.

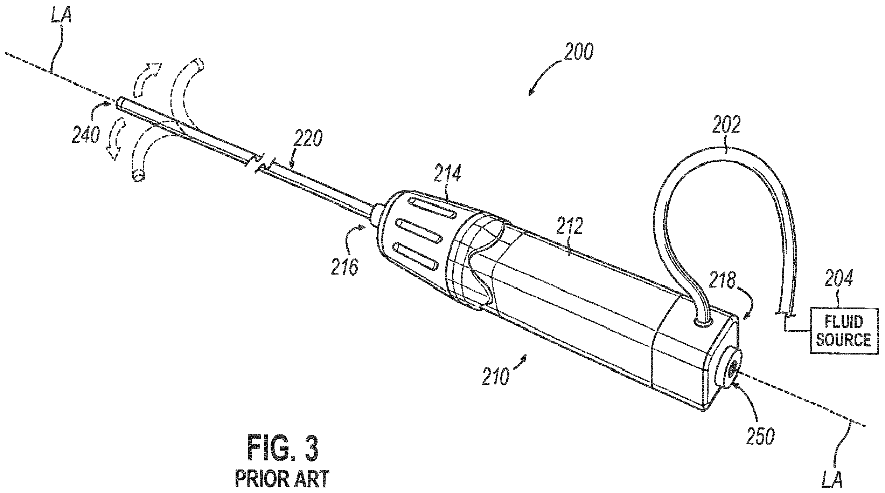

FIG. 3 shows an example of a delivery sheath (200) that may be used in such procedures. Delivery sheath (200) of this example includes a handle assembly (210) with a hollow shaft (220) extending distally from a distal end (216) of handle assembly (210). Handle assembly (210) is configured for grasping by a casing (212). The open distal end (240) of the hollow shaft (220) is operable to deflect laterally away from a longitudinal axis (LA) of the shaft. This deflection is controlled by a rotary knob (214) at distal end (216) of handle assembly (210). Rotary knob (214) is rotatable relative to casing (212), about the longitudinal axis (LA), to thereby actuate components that drive lateral deflection of open distal end (240) of hollow shaft (220). By way of example only, such actuation components may include one or more pull wires, bands, or any other suitable structures as will be apparent to those skilled in the art in view of the teachings herein.

As shown in FIG. 3, a tube (202) extends laterally from the proximal end (218) of handle assembly (210). Tube (202) of this example is in fluid communication with a hollow interior (not shown) defined within handle assembly (210), with the hollow interior being in fluid communication with the interior of hollow shaft (220). Tube (202) of the present example is further in fluid communication with a fluid source (204). By way of example only, fluid source (204) may contain saline or any other suitable fluid. In some instances, fluid from fluid source (204) is communicated through tube (202), a hollow interior region defined within handle assembly (210), and the interior of hollow shaft (220), to thereby flush the fluid path defined by tube (202), the hollow interior region defined within handle assembly (210), and the interior of hollow shaft (220).

As shown in FIG. 3, proximal end (218) of handle assembly (210) further includes an insertion port (250). Insertion port (250) is aligned with the longitudinal axis (LA) and provides a port for inserting end effector (140) and catheter (120) into hollow shaft (220) as will be described in greater detail below. In some versions, the interior of hollow shaft (220) is sized to constrain end effector (140) to the non-expanded state while end effector (140) is positioned within the hollow shaft (220). Insertion port (250) of this example includes an annular protrusion (252) defining an opening (254). Protrusion (252) protrudes proximally from casing (212) at proximal end (218). In some versions, protrusion (252) is omitted.

A seal (260) is positioned within opening (254). By way of example only, seal (260) may include an elastomeric membrane or other kind of component(s) as will be apparent to those skilled in the art in view of the teachings herein. Seal (260) of the present example further includes a slit arrangement (262) that is configured to facilitate insertion of an instrument (e.g., catheter (120) or an insert member (300) as described below, etc.) through seal (260). In the present example, slit arrangement (262) is in the form of a "+" sign, though any other suitable kind of configuration may be used. When nothing is inserted through seal (260), seal (260) is configured to provide a fluid-tight seal that prevents fluid from escaping the portion of the above-described fluid path defined within handle assembly (210) via insertion port (250); and prevents air from entering the above-described fluid path defined within handle assembly (210) via insertion port (250). When an instrument is inserted through seal (260), seal (260) still substantially maintains a fluid-tight seal of port (250), preventing fluid from escaping the above-described fluid path defined within handle assembly (210) via insertion port (250); and preventing air from entering above-described fluid path defined within handle assembly (210) via insertion port (250), while still allowing the inserted instrument to translate relative to seal (260). Thus, regardless of whether an instrument is disposed in insertion port (250), seal (260) may prevent fluids from leaking out through insertion port (250) and prevent air from being aspirated into the heart (H) of the patient (PA) via insertion port (250).

In some versions, delivery sheath (200) is configured and operable like the CARTO VIZIGO^{®} bi-directional delivery sheath by Biosense Webster, Inc. of Irvine, California.

### IV. Example of Cylindraceous Insert Member with Flared End

In some procedures, end effector (140) and catheter (120) may be inserted directly into insertion port (250) in order to enter shaft (220) and thereby exit distal end (240) of shaft (220). In some such procedures, a rigid cylindrical insert member is first inserted through seal (260) at slit arrangement (262); and end effector (140) and catheter (120) are then advanced distally through the hollow interior of the cylindrical insert member. The cylindrical insert may assist in providing initial penetration of seal (260) for end effector (140) and catheter (120), which may otherwise be rather difficult for relatively small diameter end effectors (140) and catheters (120) and/or for flexible (e.g., expandable) end effectors (140). Such a cylindrical insert member may be shaped as a pure cylinder (e.g., a straight tube with a uniform inner and outer diameter along its full length). Catheter assembly (100) may be advanced distally to a point where nozzle member (116) of handle assembly (110) reaches the proximal end of the cylindrical insert member. In such cases, the rigid proximal end of the cylindrical insert member may provide strain on the proximal end of catheter (120), which may be undesirable as the strain may compromise the structural integrity of catheter (120). Similarly, the rigid proximal end of the cylindrical insert member may encourage the formation of kinks at the proximal end of catheter (120). It may therefore be desirable to provide a version of an insert member that eliminates or otherwise reduces the risk of strain or kinking in catheter (120) at the proximal end of the insert member.

In some cases where a cylindrical insert member is used, the operator may inadvertently insert the cylindrical insert member through insertion port (250) too far, to the point where the proximal end of the cylindrical insert member passes fully through seal (260). This may be a particular risk in instances where the physician (PH) uses a catheter (120) and cylindrical insert member with a size (e.g., 8 French) that is smaller than the size of catheter and cylindrical insert member (e.g., 10 French) that delivery sheath (200) was intended to be used with. In some instances where the proximal end of the cylindrical insert member passes distally beyond seal (260), it may be difficult or impossible to remove the cylindrical insert member from handle assembly (210). In addition, or in the alternative, having an insert member jammed in seal (260) may prevent seal (260) from providing a fluid-tight seal at insertion port (250), such that air or other fluids may leak through insertion port (250). In a worst-case scenario, the cylindrical insert member may further pass through shaft (220) of delivery sheath (200) and exit distal end (216), such that the cylindrical insert member is undesirably deposited into the patient (PA). It may therefore be desirable to provide a version of an insert member that eliminates or otherwise reduces the risk of the insert member passing fully through seal (260) or other portions of insertion port (250).

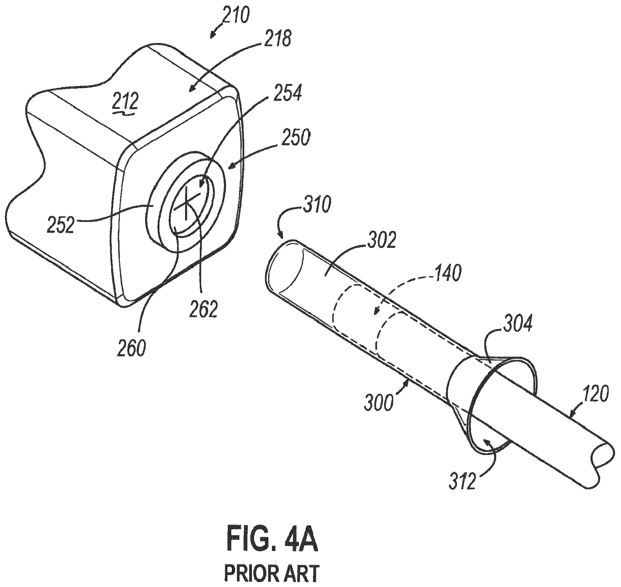

FIGS. 4A-4C show an example of an insert member (300) that may be used to assist in inserting end effector (140) and catheter (120) through insertion port (250) of delivery sheath (200). Insert member (300) of this example includes a cylindrical distal portion (302) and a flared proximal portion (304). Distal portion (302) is in the form of a straight cylinder shaft and defines a lumen (not shown) that proximally terminates at flared proximal portion (304) and distally terminates at distal end (310) of insert member (300). Proximal portion (304) has a frustoconical shape leading into the lumen and defines proximal end (312) of insert member (300). Proximal portion (304) thus tapers inwardly toward the central longitudinal axis (LA) of insert member (300) in the proximal-to-distal direction. In the present example, insert member (300) is substantially rigid.

Insert member (300) is configured to receive end effector (140) and catheter (120) while end effector (140) is in the non-expanded state, as shown in FIG. 4A. The lumen may be sized to closely complement the outer diameter of catheter (120) while permitting catheter (120) to slide freely through insert member (300). In some versions, the lumen is sized to constrain end effector (140) to the non-expanded state while end effector (140) is positioned within the lumen. The frustoconical shape of proximal portion (304) may provide a lead-in that further assists in insertion of end effector (140) and catheter (120) into proximal end (312) of insert member (300). The length of insert member (300) may be substantially less than the length of catheter (120), such that end effector (140) may protrude distally past distal end (310) of insert member (300) while insert member (300) is disposed about catheter (120).

In an example of use of insert member (300), insert member (300) may first be partially disposed about end effector (140) and the distal end of catheter (120) as shown in FIG. 4A. The combination of insert member (300), end effector (140), and catheter (120) may be positioned for insertion into insertion port (250). Thus, the longitudinal axis (LA) of catheter (120) may be aligned with the longitudinal axis (LA) of delivery sheath (200). At this stage in the present example, end effector (140) is longitudinally disposed between distal end (310) of insert member (300) and proximal end (312) of insert member (300). As noted above, end effector (140) may be constrained by the lumen to the non-expanded state at this stage.

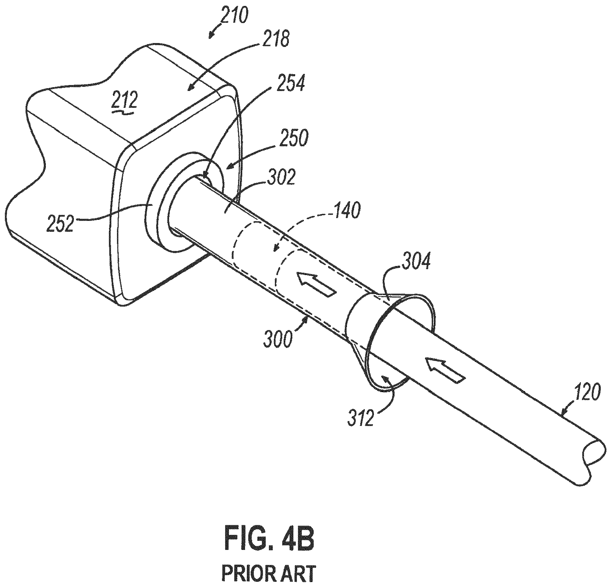

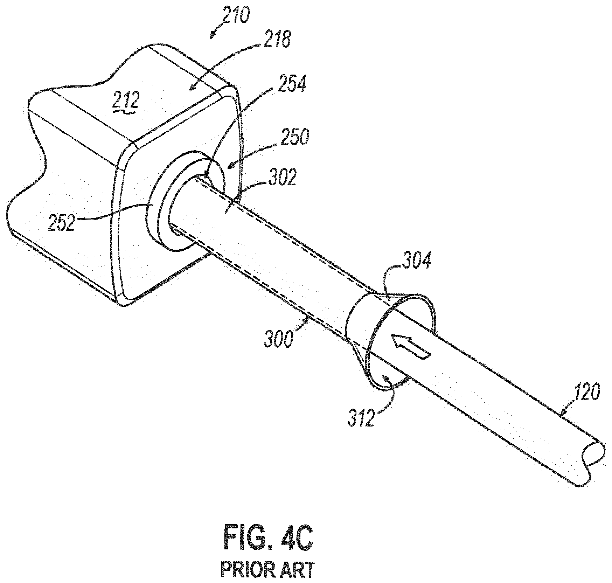

Next, the physician may advance the combination of insert member (300), end effector (140), and catheter (120) distally toward insertion port (250), such that distal end (310) of insert member (300) penetrates seal (260) at slit arrangement (262), as shown in FIG. 4B. In this example, distal end (310) of insert member (300) passes through seal (260) before end effector (140) is advanced distally beyond distal end (310) of insert member (300). Once distal end (310) of insert member (300) has passed through seal (260), catheter (120) is advanced such that end effector (140) is advanced distally beyond distal end (310) of insert member (300), as shown in FIG. 4C. As catheter (120) is advanced, end effector (140) and catheter (120) pass distally through the interior of shaft (220). End effector (140) may be constrained by the interior of shaft (220) to the non-expanded state at this stage. End effector (140) eventually reaches a point where end effector (140) is distal to distal end (240) of shaft (220). End effector (140) may no longer be constrained to the non-expanded state at this stage, such that end effector (140) may be selectively transitioned to the expanded state. In some versions of the procedure, after reaching the state shown in FIG. 4C, insert member (300) is retracted proximally relative to catheter (120) as the physician (PH) continues to advance catheter (120) distally. In other words, distal end (310) of insert member (300) may be proximal to insertion port (250) during at least part of the procedure where catheter (120) is advanced distally into delivery sheath (200).

In some scenarios, during normal operation of catheter assembly (100) and delivery sheath (200), when end effector (140) is positioned distally relative to distal end (240) of shaft (220), nozzle member (116) of handle assembly (110) and insert member (300) are both spaced proximally away from insertion port (250). Thus, some versions of catheter assembly (100), delivery sheath (200), and insert member (300) may be configured to allow end effector (140) to be distally exposed from shaft (220) and thus operated within the heart (H) of the patient (PA), without insert member (300) needing to contact insertion port (250); and without nozzle member (116) needing to contact insert member (300). In such scenarios, insert member (300) may simply be positioned about a region of catheter (120) that is longitudinally interposed between insertion port (250) and nozzle member (116) of handle assembly (110).

In the event that the physician (PH) continues to advance catheter assembly (100) distally to the point where nozzle member (116) of handle assembly (110) engages insert member (300), flared proximal portion (304) of insert member (300) may eventually engage annular protrusion (252) of insertion port (250). While cylindrical distal portion (302) of insert member (300) has an outer diameter that is smaller than the diameter of opening (254), flared proximal portion (304) of insert member (300) has an outer diameter that is larger than the diameter of opening (254). Thus, flared proximal portion (304) of insert member (300) will engage annular protrusion (252) of insertion port (250), and this interaction between flared proximal portion (304) of insert member (300) and annular protrusion (252) of insertion port (250) will arrest insert member (300) and thereby prevent insert member (300) from advancing further distally into insertion port (250).

While flared proximal portion (304) of insert member (300) engages annular protrusion (252) of insertion port (250) in the foregoing example, other configurations may provide engagement between flared proximal portion (304) and seal (260). In such scenarios, annular protrusion (252) may simply be absent. Alternatively, flared proximal portion (304) may have an outer diameter that is sized to pass through the opening defined by annular protrusion (252) but not through seal (260). In either case, slit arrangement (262) may be configured to permit cylindrical distal portion (302) of insert member (300) through seal (260) but prevent flared proximal portion (304) of insert member (300) from passing through seal (260). As another merely illustrative alternative, insertion port (250) may include some other structure that engages annular protrusion (252) and thereby arrests insertion of insert member (300) through insertion port (250).

In addition to arresting distal insertion of insert member (300) into insertion port (250), flared proximal portion (304) of insert member (300) may further eliminate or otherwise reduce strain that might otherwise occur at the junction of the proximal end (312) of insert member (300) and catheter (120) by providing greater freedom of catheter (120) to deflect laterally relative to proximal end (312) of insert member (300).

In some variations of the procedure described above, insert member (300) is inserted into insertion port (250) before end effector (140) and catheter (120) are inserted into insert member (300). In other words, end effector (140) and catheter (120) may be fully decoupled from insert member (300) when insert member (300) is initially inserted into insertion port (250). In some such variations of the procedure, insert member (300) is first fully inserted into insertion port (250), to the point where flared proximal portion (304) of insert member (300) engages annular protrusion (252) of insertion port (250), before end effector (140) and catheter (120) are inserted into insert member (300).

### V. Examples of Insert Devices with Coupling Members

In some scenarios, it may be desirable to secure an insert device like insert member (300) to a sheath handle assembly like handle assembly (210), with a longitudinal axis of insert member (300) aligned with a longitudinal axis of insertion port (250) and thus with the longitudinal axis (LA) of delivery sheath (200), such as for centering the distal end of catheter (120) relative to slit arrangement (262) of seal (260) when catheter (120) is advanced through insert member (300), and/or for orienting the longitudinal axis of catheter (120) orthogonally relative to a plane defined by seal (260) when catheter (120) is advanced through insert member (300). Such securing of insert member (300) to handle assembly (210) may promote insertion of the distal end of catheter (120) through seal (260) at a desired position and orientation relative to seal (260) that may minimize the amount of force required to push the distal end of catheter (120) through seal (260) and inhibit damage to seal (260) and/or the distal end of catheter (120) including end effector (140). In addition, or alternatively, such securing of insert member (300) to handle assembly (210) may allow the physician (PH) or other operator to release insert member (300) after securing insert member (300) to handle assembly (210), thereby enabling the operator to hold handle assembly (210) in one of the operator's hands (e.g., without having to simultaneously hold insert member (300) with that hand) while inserting catheter (120) through seal (260) with the operator's other hand. In view of the foregoing, it may be desirable to add a coupling member to an insert member (300) to facilitate such securing of insert member (300) to handle assembly (210). Examples of various forms that such coupling members may take will be described in greater detail below.

While the examples described below are provided in the context of inserting catheter (120) through seal (260) and into delivery sheath (200), the teachings herein may be readily applied to other contexts, such as those where some other kind of instrument (e.g., a dilator, another kind of non-catheter instrument, etc.) is inserted into delivery sheath (200).

### A. Example of Cylindraceous Insert Member with Threaded Distal Cap Portion

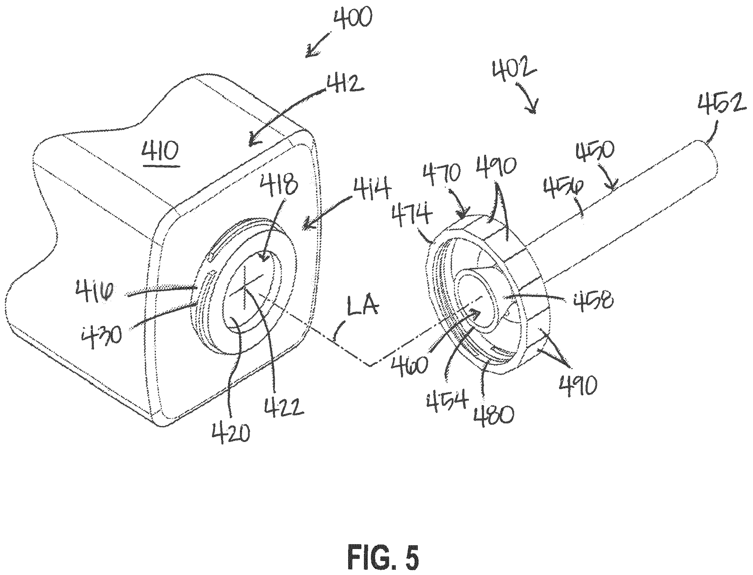

FIGS. 5-6D show a proximal portion of another example of a handle assembly (400) that may be incorporated into delivery sheath (200) in place of handle assembly (210), and an example of an insert member (402) that may be selectively secured to handle assembly (400) and used in a manner similar to that described above for insert member (300). Handle assembly (400) and insert member (402) are similar to handle assembly (210) and insert member (300) described above, respectively, except as otherwise described below. Handle assembly (400) of this example is configured for grasping by a casing (410) and includes a proximal end (412) and a distal end (not shown). Proximal end (412) has an insertion port (414), which defines a longitudinal axis (LA) and includes an annular protrusion (416) defining an opening (418) and protruding proximally from casing (410) at proximal end (412). A seal (420) is positioned within opening (418) and includes a slit arrangement (422) that is configured to facilitate insertion of an instrument (e.g., catheter (120), etc.) through seal (420). In the present example, slit arrangement (422) is in the form of a "+" sign, though any other suitable kind of configuration may be used.

As shown, insertion port (414) further includes a thread in the form of a helical ridge (430) extending radially outwardly from a generally cylindrical outer surface of annular protrusion (416) for threadably engaging a corresponding portion of insert member (402), as described in greater detail below. In some versions, ridge (430) may also be configured to threadably engage a corresponding portion of a balloon dilation catheter assembly (not shown), such as a dilator cap of the HELIOSTAR^{™} balloon ablation catheter by Biosense Webster, Inc. of Irvine, California. In this regard, handle assembly (400) may be configured similarly to that of the GUIDESTAR^{™} introducer sheath by Biosense Webster, Inc. of Irvine, California.

Insert member (402) of this example includes a tube (450) extending longitudinally between a proximal end (452) and a distal end (454). Tube (450) has a cylindrical main portion (456) and a flared distal portion (458). Main portion (456) is in the form of a straight cylinder while distal portion (458) has a frustoconical shape such that distal portion (458) tapers radially outwardly from main portion (456) to distal end (454) of tube (450). Main portion (456) and distal portion (458) collectively define a lumen (460) that extends longitudinally between proximal and distal ends (452, 454) of tube (450) and that is sized to closely complement the outer diameter of catheter (120) while permitting catheter (120) to slide freely through tube (450). Lumen (460) may be sized to constrain end effector (140) to the non-expanded state while end effector (140) is positioned within lumen (460). In the present example, tube (450) is substantially rigid. In some variations, proximal end (452) and distal portion (458) are both flared. In some other variations, proximal end (452) is flared but distal portion (458) is not flared.

Insert member (402) of the present example further includes a coupling member in the form of a distal cap (470) arranged coaxially with tube (450). Cap (470) includes a generally flat and/or dome-shaped proximal end wall (472) extending radially outwardly relative to main portion (456) of tube (450) at or near the interface between main and distal portions (456, 458), such as at a position slightly proximal of distal portion (458), and a generally annular distal sidewall (474). In the example shown, sidewall (474) of cap (470) extends circumferentially about distal portion (458) such that a generally cylindrical inner surface of sidewall (474) confronts a generally frustoconical outer surface of distal portion (458), with a clearance gap between the generally cylindrical inner surface of sidewall (474) and the generally frustoconical outer surface of distal portion (458) that is sufficiently large to receive annular protrusion (416) of insertion port (414).

Cap (470) of the present example further includes a thread in the form of a helical groove (480) extending radially outwardly from the generally cylindrical inner surface of sidewall (474) for threadably engaging ridge (430) of insertion port (414). Groove (480) may extend along a helical path similar to that of ridge (430) for promoting the threadable engagement between groove (480) and ridge (430). It will be appreciated that the threads of insertion port (414) and cap (470) may be of any suitable form for threadably engaging each other and are not limited to the particular configurations of ridge (430) and groove (480) shown. For example, while ridge (430) is provided on insertion port (414) and groove (480) is provided on cap (470) in the present version, in some other versions ridge (430) may be provided on cap (470) and groove (480) may be provided on insertion port (414).

The threadable engagement between ridge (430) and groove (480) may facilitate secure coupling of insert member (402) to handle assembly (400), with a longitudinal axis of insert member (402) and, more particularly, a longitudinal axis of lumen (460) aligned with the longitudinal axis (LA) of insertion port (414). In this manner, lumen (460) may be centered relative to slit arrangement (422) of seal (420) and may be oriented orthogonally relative to a plane defined by seal (420) when insert member (402) is secured to handle assembly (400). In some versions, distal end (454) of tube (450) may be configured to abut a proximal surface of seal (420) when insert member (402) is secured to handle assembly (400). In this regard, the flared configuration of distal portion (458) of tube (450) may permit distal end (454) to pass into opening (418) of annular protrusion (416) of insertion port (414); but may prevent distal end (454) from being inserted through seal (420). For example, distal portion (458) may have an outer diameter selected to permit distal end (454) to pass into opening (418) of annular protrusion (416) of insertion port (414) in cases where delivery sheath (200) is configured for use with catheter (120) having a size on the French catheter scale of between about 13.5 Fr and about 14 Fr, for example. In some other versions, the flared configuration of distal portion (458) of tube (450) may prevent distal end (454) from being inserted into insertion port (414).

In the example shown, sidewall (474) of cap (470) further includes a plurality of flat outer surfaces (490) configured to be gripped by the physician (PH) or other operator for assisting the operator with rotating cap (470) relative to handle assembly (400) to threadably engage groove (480) with ridge (430) and/or to threadably disengage groove (480) from ridge (430). Cap (470) of the present example also includes at least one vent or bleeder hole (492) extending through end wall (472) for venting fluid (e.g., air) from a space between a distal surface of end wall (472) of cap (470) and handle assembly (400) to an exterior of cap (470) when cap (470) is secured to handle assembly (400).

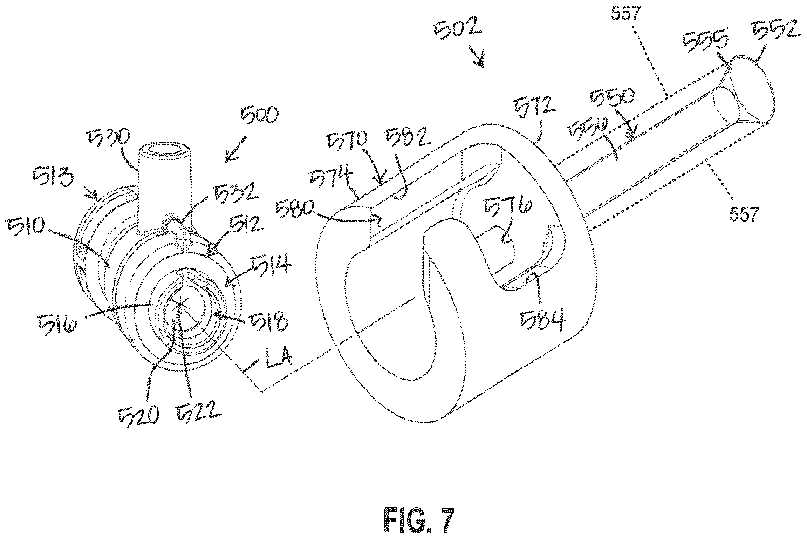

Insert member (402) may be constructed of any suitable translucent (e.g., transparent) or opaque material, such as a polymeric material (e.g., plastic) or a metallic material. Other suitable materials that may be used to form insert member (402) will be apparent to those skilled in the art in view of the teachings herein. In the example shown, tube (450) and cap (470) are integrally formed together with each other as a unitary (e.g., monolithic) piece to define insert member (402). In this regard, insert member (402) may be manufactured via 3D printing, injection molding, investment casting, machining, and/or any other suitable manufacturing techniques. In other versions, tube (450) and cap (470) are individually formed separately from each other as discrete parts and coupled to each other to define insert member (402), such that insert member (402) may be referred to as an assembly. It will be appreciated that tube (450) and cap (470) may be constructed of the same or different materials. While tube (450) of the present example is fixed against movement relative to cap (470), tube (450) may alternatively be movable relative to cap (470). For example, tube (450) may be slidably coupled to cap (470) to facilitate longitudinal sliding of tube (450) relative to cap (470), such as in a manner similar to any of those described below in connection with FIGS. 7-14.

While distal portion (458) of tube (450) is flared in the present example, distal portion (458) may alternatively be cylindrical. For example, distal portion (458) may be in the form of a straight cylinder having inner and outer dimensions similar to those of main portion (456). Such a configuration of distal portion (458) may permit distal end (454) to be inserted through seal (420) in a manner similar to that described above in connection with FIGS. 4A-4C and/or any of those described below in connection with FIGS. 7-14. In some such cases, tube (450) may include a flared proximal portion (not shown) having a frustoconical shape such that the proximal portion tapers radially outwardly from main portion (456) to proximal end (452) of tube (450) and cooperating with main portion (456) to define lumen (460). Such a flared proximal portion may provide a lead-in for assisting with insertion of end effector (140) and catheter (120) into proximal end (452) of tube (450). In some other versions, tube (450) may include both flared distal portion (458) and such a flared proximal portion.

While not shown, insert member (402) may include any one or more of a seal member, enhanced structural support features such as ribs, torque-driving features such as external fins, and/or catheter-gripping features such as internal fins. By way of example only, insert member (402) may be configured and operable in accordance with at least some of the teachings of U.S. Pub. No. 2021/0113812, entitled "Flared Insert Member for Use with Catheter Assembly," published April 22, 2021, the disclosure of which is incorporated by reference herein, in its entirety.

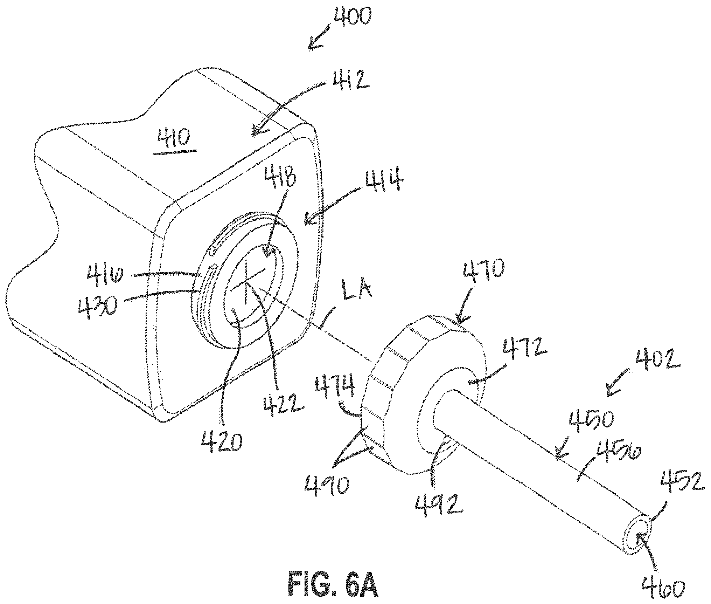

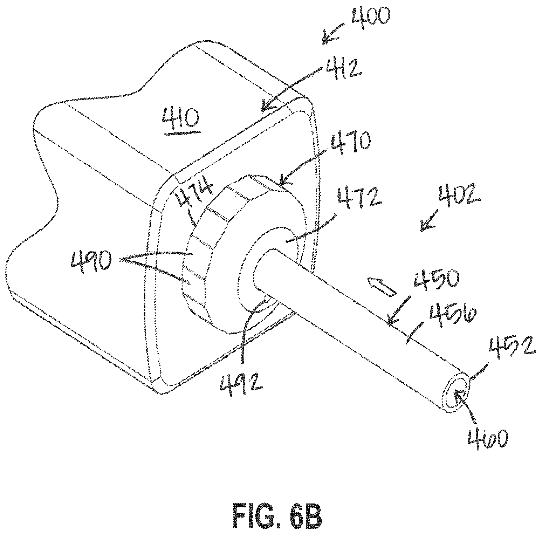

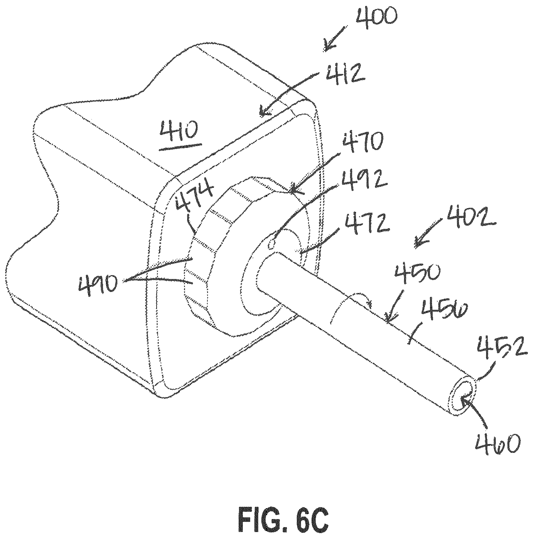

In an example of use of insert member (402), the longitudinal axis (LA) of insert member (402) may be aligned with the longitudinal axis (LA) of insertion port (414), as shown in FIG. 6A. Next, the physician (PH) may advance insert member (402) distally toward insertion port (414), such that annular protrusion (416) of insertion port (414) is at least partially received between the generally cylindrical inner surface of sidewall (474) of cap (470) and the generally frustoconical outer surface of distal portion (458) of tube (450), as shown in FIG. 6B. The physician (PH) may then rotate cap (470) relative to handle assembly (400) to threadably engage groove (480) of cap (470) with ridge (430) of insertion port (414) and thereby secure insert member (402) to handle assembly (400), as shown in FIG. 6C.

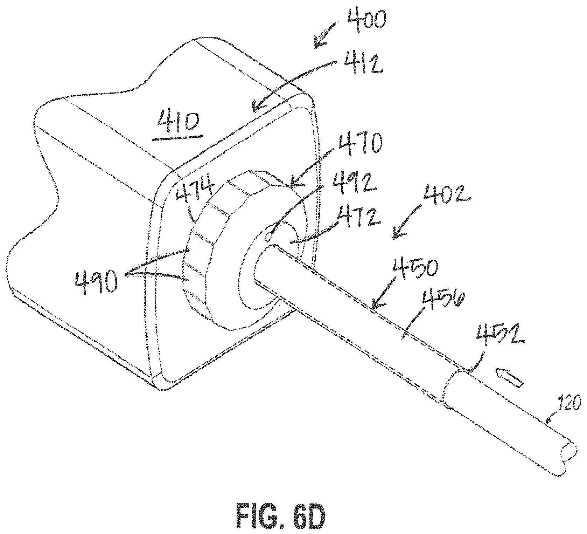

When insert member (402) is in the secured state shown in FIG. 6C, the longitudinal axis of lumen (460) is aligned with the longitudinal axis (LA) of insertion port (414), such that lumen (460) is centered relative to slit arrangement (422) of seal (420) and oriented orthogonally relative to the plane defined by seal (420), and distal end (454) of tube (450) abuts the proximal surface of seal (420). It will be appreciated that seal (420) may remain unpenetrated during securing of insert member (402) to handle assembly (400), such that the securing of insert member (402) to handle assembly (400) may be performed before inserting end effector (140) and catheter (120) into insert member (402). Once insert member (402) has been secured to handle assembly (400), catheter (120) is slid through lumen (460) of insert member (402) and advanced distally beyond distal end (454) to penetrate seal (420) at slit arrangement (422) such that end effector (140) and catheter (120) may pass distally through the interior of shaft (220), as shown in FIG. 6D. End effector (140) eventually reaches a point where end effector (140) is distal to distal end (240) of shaft (220) such that end effector (140) may be selectively transitioned to the expanded state.

### B. Example of Insert Member Assembly with Distal Cap Having L-Shaped Bayonet Slot

FIGS. 7-8D show an example of a sheath hub (500) for another handle assembly that may be incorporated into delivery sheath (200) in place of handle assembly (210), and an example of an insert member assembly (502) that may be selectively secured to sheath hub (500) and used in a manner similar to that described above for insert member (300). Sheath hub (500) may be incorporated into a handle assembly similar to handle assembly (210) and insert member assembly (502) is similar to insert member (402) described above, except as otherwise described below. In particular, sheath hub (500) may extend perpendicularly from the handle assembly and may be rigidly secured relative to the housing of the handle assembly, thereby providing a mechanical ground for insert member assembly (502) relative to the handle assembly of the delivery sheath. Sheath hub (500) of this example has a body (510) that is configured to be coupled to a casing (not shown) similar to casing (410) and includes a proximal end (512) and a distal end (513). Proximal end (512) has an insertion port (514), which defines a longitudinal axis (LA) and includes an annular protrusion (516) defining an opening (518) and protruding proximally from body (510) at proximal end (512). A seal (520) is positioned within opening (518) and includes a slit arrangement (522) that is configured to facilitate insertion of an instrument (e.g., catheter (120), etc.) through seal (520). In the present example, slit arrangement (522) is in the form of an "*" symbol, though any other suitable kind of configuration may be used.

As shown, sheath hub (500) further includes a protrusion in the form of a sideport (530) extending radially outwardly from body (510) near proximal end (512) for engaging a corresponding portion of insert member assembly (502), as described in greater detail below. In the example shown, sheath hub (500) also includes an elongate tab (532) extending radially outwardly from body (510) between sideport (530) and proximal end (512). In some versions, sideport (530) may be configured to be selectively fluidly coupled with a fluid source (not shown). Such a fluid source may contain saline, a fluoroscopy contrast fluid, and/or any other suitable kind of fluid. Fluid from the fluid source may reach hollow shaft (220) via sideport (530); and may ultimately be expelled from open distal end (240) of hollow shaft (220). By way of further example only, sheath hub (500) may be configured similarly to that of the CARTO VIZIGO^{®} introducer sheath by Biosense Webster, Inc. of Irvine, California.

Insert member assembly (502) of this example includes a tube (550) extending longitudinally between a proximal end (552) and a distal end (554). Tube (550) has a flared proximal portion (555) and a cylindrical main portion (556). Main portion (556) is in the form of a straight cylinder while proximal portion (555) has a frustoconical shape such that proximal portion (555) tapers radially outwardly from main portion (556) to proximal end (552) of tube (550). Main portion (556) and proximal portion (555) collectively define a lumen (560) that extends longitudinally between proximal and distal ends (552, 554) of tube (550) and that is sized to closely complement the outer diameter of catheter (120) while permitting catheter (120) to slide freely through tube (550). Lumen (560) may be sized to constrain end effector (140) to the non-expanded state while end effector (140) is positioned within lumen (560). In the present example, tube (550) is substantially rigid. In some versions, the rigidity of tube (550) may be enhanced by providing thickened sidewalls, as indicated by broken lines (557) in FIG. 7. In some variations, proximal end (552) and distal end (554) are both flared. In some other variations, distal end (554) is flared but proximal end (552) is not flared. Proximal end (552) and/or distal end (554) may still provide a conical lead-in/lead-out in versions where tube has thickened sidewalls as indicated by broken lines (557).

Insert member assembly (502) of the present example further includes a coupling member in the form of a distal cap (570) arranged coaxially with tube (550). Cap (570) includes a generally flat proximal end wall (572) extending radially outwardly relative to main portion (556) of tube (550), and a generally cylindrical distal sidewall (574). In the example shown, cap (570) includes a central bore (576) extending longitudinally through end wall (572) and configured to slidably receive main portion (556) of tube (550) to facilitate longitudinal sliding of tube (550) relative to cap (570). For example, tube (550) may be configured to slide longitudinally relative to cap (570) between a first position at which cap (570) is at or near distal end (554) of tube (550), and a second position at which cap (570) is engaged with proximal portion (555) of tube (550), as described in greater detail below. In this regard, main portion (556) of tube (550) may have an outer diameter that is at least slightly smaller than a diameter of central bore (576) to permit such longitudinal sliding, while proximal portion (555) of tube (550) may have an outer diameter that is larger than the diameter of central bore (576). In some versions, a gasket (not shown) may be disposed between central bore (576) and main portion (556) of tube (550) for providing a fluid-tight seal therebetween while permitting such longitudinal sliding. In addition, or alternatively, a keyway (not shown) may extend radially outwardly from central bore (576) for receiving a corresponding key (not shown) extending radially outwardly from main portion (556) of tube (550) to fix tube (550) and cap (570) against rotation relative to each other about a longitudinal axis of insert member assembly (502) while permitting such longitudinal sliding. In still other variations tube (550) is rigidly secured relative to cap (570), such that tube (550) is not slidable relative to cap (570).

In the example shown, sidewall (574) of cap (570) extends circumferentially about a region of main portion (556) such that a generally cylindrical inner surface of sidewall (574) confronts a generally cylindrical outer surface of main portion (556), with a clearance gap between the generally cylindrical inner surface of sidewall (574) and the generally cylindrical outer surface of main portion (556) that is sufficiently large to receive body (510) of sheath hub (500). In this regard, the generally cylindrical inner surface of sidewall (574) may be sized to closely complement an outer diameter of body (510) of sheath hub (500) while permitting body (510) of sheath hub (500) be selectively inserted into and removed from an interior of cap (570).

Cap (570) of the present example further includes a bayonet feature in the form of a generally L-shaped slot (580) extending radially outwardly from the generally cylindrical inner surface of sidewall (574) to a generally cylindrical outer surface of sidewall (574) for engaging sideport (530) of sheath hub (500) in a bayonet-style manner. While a single slot (580) is shown, other versions may include any other suitable number of slots (580) such as for allowing a particular slot (580) to be selected for engaging sideport (530). In the example shown, slot (580) includes a straight distal entryway portion (582) extending proximally from a distal end of cap (570), and a closed-ended proximal locking portion (584) extending orthogonally (e.g., at about 90°) from entryway portion (582) (e.g., in a clockwise direction within the frame of reference of FIG. 7) for promoting the bayonet-style engagement between slot (580) and sideport (530). For example, entryway portion (582) may be configured to receive sideport (530) and to be guided therealong until sideport (530) reaches a proximal end of entryway portion (582); and locking portion (584) may be configured to then receive sideport (530) and to be guided therealong until sideport (530) is sufficiently captured within locking portion (584) to inhibit longitudinal movement of cap (570) and sheath hub (500) relative to each other (e.g., to inhibit proximal translation of cap (570) relative to sheath hub (500)) in the absence of an accompanying rotational force applied between cap (570) and sheath hub (500).

It will be appreciated that the bayonet features of sheath hub (500) and cap (570) may be of any suitable form for engaging each other in a bayonet-style manner and are not limited to the particular configurations of sideport (530) and slot (580) shown. For example, while sideport (530) is configured to engage slot (580) in a bayonet-style manner in the present version, in some other versions sheath hub (500) may include a separate protrusion extending radially outwardly from body (510) and configured to engage slot (580) in a bayonet-style manner.

The bayonet-style engagement between sideport (530) or any other such protrusion and slot (580) may facilitate secure coupling of cap (570) to sheath hub (500), with a longitudinal axis of insert member assembly (502) and, more particularly, a longitudinal axis of lumen (560) aligned with the longitudinal axis (LA) of insertion port (514). In this manner, lumen (560) may be centered relative to slit arrangement (522) of seal (520) and may be oriented orthogonally relative to a plane defined by seal (520) when cap (570) is secured to sheath hub (500). In some versions, distal end (554) of tube (550) may be configured to be inserted through seal (520) when cap (570) is secured to sheath hub (500). In this regard, the cylindrical configuration of main portion (556) of tube (550) may permit distal end (554) to be inserted through seal (520), and the slidable coupling of main portion (556) of tube (550) to cap (570) may permit such insertion of distal end (554) through seal (520) to be performed while cap (570) remains secured to sheath hub (500). In addition, or alternatively, the flared configuration of proximal portion (555) of tube (550) may provide a lead-in for assisting with insertion of end effector (140) and catheter (120) into proximal end (552) of tube (550), and/or may enable proximal portion (555) to engage a proximal surface of end wall (572) of cap (570) and thereby limit distal advancement of tube (550) into insertion port (514). While not shown, cap (570) may also include at least one vent or bleeder hole extending through end wall (572) for venting fluid (e.g., air) from a space between a distal surface of end wall (572) of cap (570) and sheath hub (500) to an exterior of cap (570) when cap (570) is secured to sheath hub (500).

Tube (550) and cap (570) of insert member assembly (502) may each be constructed of any suitable translucent (e.g., transparent) or opaque material, such as a polymeric material (e.g., plastic) or a metallic material. Other suitable materials that may be used to form tube (550) and cap (570) of insert member assembly (502) will be apparent to those skilled in the art in view of the teachings herein. In the example shown, tube (550) and cap (570) are individually formed separately from each other as discrete parts and coupled to each other via central bore (576) to define insert member assembly (502). In this regard, tube (550) and cap (570) may each be manufactured via 3D printing, injection molding, investment casting, machining, and/or any other suitable manufacturing techniques. While tube (550) of the present example is movable relative to cap (570), tube (550) may alternatively be fixed against movement relative to cap (570). For example, distal end (554) of tube (550) may be configured to abut a proximal surface of seal (520) when cap (570) is secured to sheath hub (500), such as in a manner similar to that described above in connection with FIGS. 5-6D.

While proximal portion (555) of tube (550) is flared in the present example, proximal portion (555) may alternatively be cylindrical. For example, proximal portion (555) may be in the form of a straight cylinder having inner and outer dimensions similar to those of main portion (556). In some such cases, tube (550) may include a flared distal portion (not shown) having a frustoconical shape such that the distal portion tapers radially outwardly from main portion (556) to distal end (554) of tube (550) and cooperating with main portion (556) to define lumen (560). Such a flared distal portion may permit distal end (554) to pass into opening (518) of annular protrusion (516) of insertion port (514); but may prevent distal end (554) from being inserted through seal (520). Alternatively, such a flared distal portion may prevent distal end (554) from being inserted into insertion port (514). In some other versions, tube (550) may include both flared proximal portion (555) and such a flared distal portion.

While not shown, tube (550) of insert member assembly (502) may include any one or more of a seal member, enhanced structural support features such as ribs, torque-driving features such as external fins, and/or catheter-gripping features such as internal fins. By way of example only, tube (550) of insert member assembly (502) may be configured and operable in accordance with at least some of the teachings of U.S. Pub. No. 2021/0113812, entitled "Flared Insert Member for Use with Catheter Assembly," published April 22, 2021, the disclosure of which is incorporated by reference herein, in its entirety.

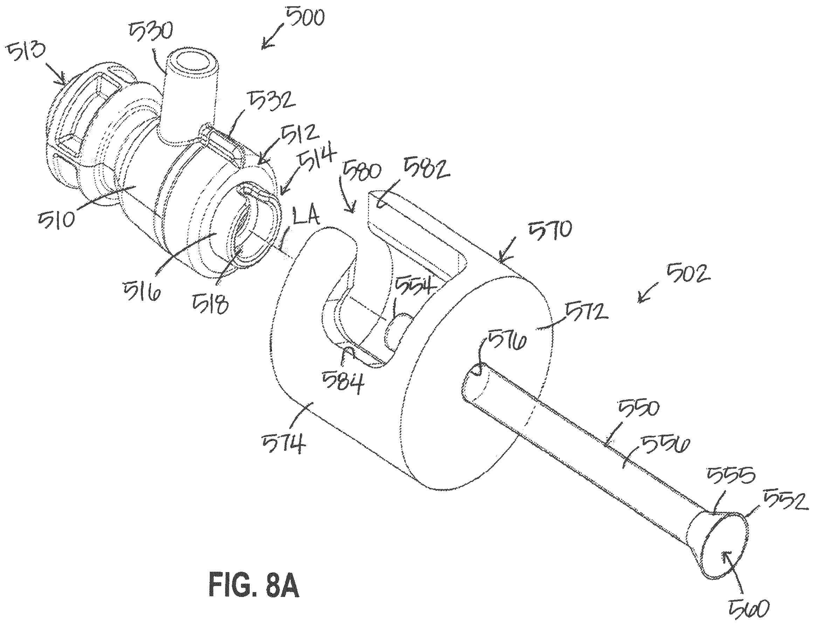

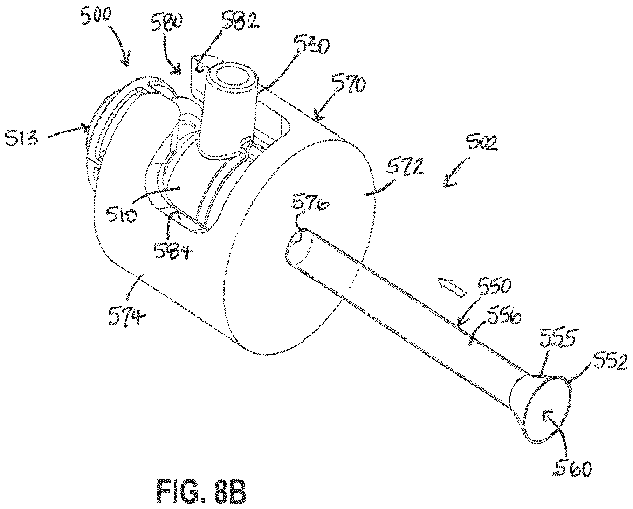

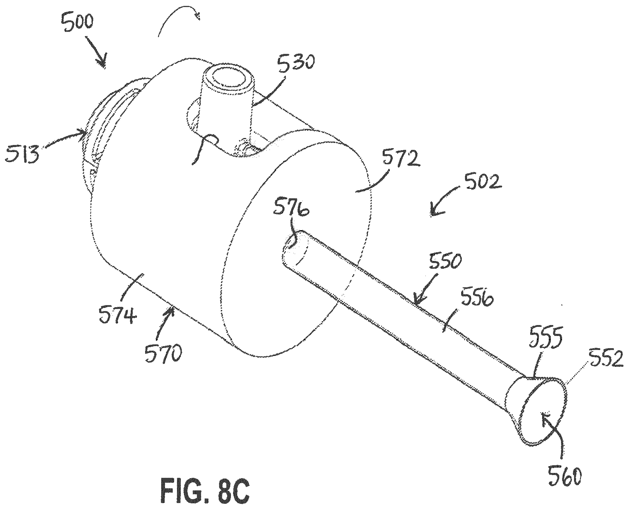

In an example of use of insert member assembly (502), the longitudinal axis (LA) of insert member assembly (502) may be aligned with the longitudinal axis (LA) of insertion port (514), and entryway portion (582) of slot (580) may be aligned with sideport (530), as shown in FIG. 8A. Next, the physician (PH) may advance insert member assembly (502) distally toward insertion port (514), such that body (510) of sheath hub (500) is at least partially received between the generally cylindrical inner surface of sidewall (574) of cap (570) and the generally cylindrical outer surface of main portion (556) of tube (550), and such that sideport (530) of sheath hub (500) is at least partially received within entryway portion (582) of slot (580), as shown in FIG. 8B. The physician (PH) may then rotate cap (570) relative to sheath hub (500) to engage slot (580) of cap (570) with sideport (530) of sheath hub (500) in a bayonet-style manner by capturing sideport (530) within locking portion (584) of slot (580) and thereby secure cap (570) to sheath hub (500), as shown in FIG. 8C.

When cap (570) is in the secured state shown in FIG. 8C, the longitudinal axis of lumen (560) is aligned with the longitudinal axis (LA) of insertion port (514), such that lumen (560) is centered relative to slit arrangement (522) of seal (520) and oriented orthogonally relative to the plane defined by seal (520). When cap (570) is initially placed into the secured state, distal end (554) of tube (550) may either abut the proximal surface of seal (520) or be positioned proximally thereof to enable seal (520) to remain unpenetrated during securing of cap (570) to sheath hub (500), such that the securing of cap (570) to sheath hub (500) may be performed before inserting end effector (140) and catheter (120) into insert member assembly (502). Once cap (570) has been secured to sheath hub (500), catheter (120) may be slid through lumen (560) of insert member assembly (502), and catheter (120) and/or tube (550) may be advanced distally such that end effector (140) and/or distal end (554) penetrate seal (520) at slit arrangement (522) in a manner similar to that described above in connection with FIGS. 4A-4C.

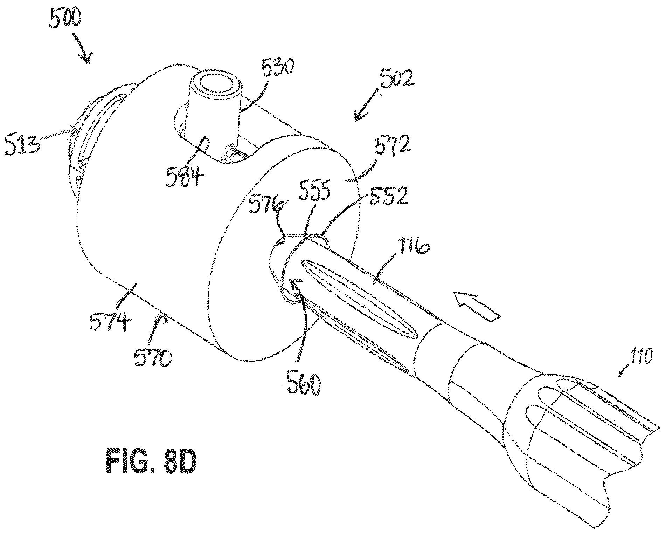

In the event that the physician (PH) continues to advance catheter assembly (100) distally to the point where nozzle member (116) of handle assembly (110) engages insert member assembly (502), flared proximal portion (555) of tube (550) may eventually engage end wall (572) of cap (570), as shown in FIG. 8D. As noted above, while cylindrical main portion (556) of tube (550) has an outer diameter that is at least slightly smaller than the diameter of central bore (576), flared proximal portion (555) of tube (550) has an outer diameter that is larger than the diameter of central bore (576). Thus, flared proximal portion (555) of tube (550) will engage the periphery of central bore (576), and this interaction between flared proximal portion (555) of tube (550) and the periphery of central bore (576)) will arrest tube (550) and thereby prevent tube (550) from advancing further distally into insertion port (514).

While tube (550) is shown in FIGS. 8C-8D as being slidable relative to cap (570), some variations may provide a fixed relationship between tube (550) and cap (570), such that tube (550) need not necessarily be slidable relative to cap (570) in all versions. For instance, tube (550) and cap (570) may be integrally formed together with each other as a unitary (e.g., monolithic) piece to define insert member assembly (502). In this regard, insert member assembly (502) may be manufactured via 3D printing, injection molding, investment casting, machining, and/or any other suitable manufacturing techniques. In other versions, tube (550) and cap (570) are individually formed separately from each other as discrete parts and coupled to each other to form insert member assembly (502). Alternatively, tube (550) and cap (570) may have any other suitable relationships with each other and may be formed and/or coupled together using any other suitable techniques.

### C. Example of Insert Member Assembly with Distal Cap Having J-Shaped Bayonet Slot

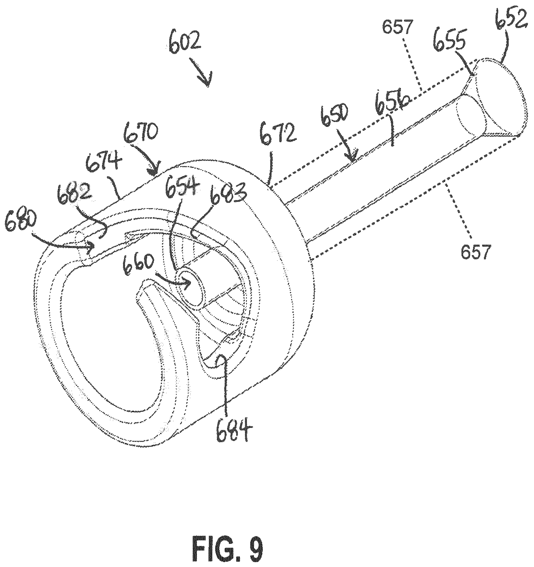

FIGS. 9-10D show another example of an insert member assembly (602) that may be selectively secured to sheath hub (500) and used in a manner similar to that described above for insert member (300), such that insert member assembly (602) may be selectively coupled with delivery sheath (200) via sheath hub (500). Insert member assembly (602) is similar to insert member assembly (502) described above, except as otherwise described below. In this regard, insert member assembly (602) of this example includes a substantially rigid tube (650) extending longitudinally between a proximal end (652) and a distal end (654). In some versions, the rigidity of tube (650) may be enhanced by providing thickened sidewalls, as indicated by broken lines (657) in FIG. 9. Tube (650) has a flared proximal portion (655) and a cylindrical main portion (656), which collectively define a lumen (660) that is sized to closely complement the outer diameter of catheter (120) while permitting catheter (120) to slide freely through tube (650). Lumen (660) may be sized to constrain end effector (140) to the non-expanded state while end effector (140) is positioned within lumen (660). In some variations, proximal end (652) and distal end (654) are both flared. In some other variations, distal end (654) is flared but proximal end (652) is not flared. Proximal end (652) and/or distal end (654) may still provide a conical lead-in/lead-out in versions where tube has thickened sidewalls as indicated by broken lines (657).

Insert member assembly (602) of the present example further includes a coupling member in the form of a distal cap (670) having a generally dome-shaped proximal end wall (672) and a generally cylindrical distal sidewall (674). In the example shown, cap (670) includes a central bore (676) extending longitudinally through end wall (672) and configured to slidably receive main portion (656) of tube (650) to facilitate longitudinal sliding of tube (650) relative to cap (670). In still other variations tube (650) is rigidly secured relative to cap (670), such that tube (650) is not slidable relative to cap (670).

Cap (670) of the present example further includes a bayonet feature in the form of a generally J-shaped slot (680) extending radially outwardly from a generally cylindrical inner surface of sidewall (674) to a generally cylindrical outer surface of sidewall (674) for engaging sideport (530) of sheath hub (500) in a bayonet-style manner. While a single slot (680) is shown, other versions may include any other suitable number of slots (680) such as for allowing a particular slot (680) to be selected for engaging sideport (530). In the example shown, slot (680) includes a straight entryway portion (682) extending proximally from a distal end of cap (670), an intermediate portion (683) extending proximally and obliquely from entry portion (682) (e.g., in a clockwise direction within the frame of reference of FIG. 9), and a closed-ended locking portion (684) extending at least slightly distally and obliquely from intermediate portion (683) (e.g., in a clockwise direction within the frame of reference of FIG. 9) for promoting the bayonet-style engagement between slot (680) and sideport (530). For example, entryway portion (682) may be configured to receive sideport (530) and to be guided therealong until sideport (530) reaches a proximal end of entryway portion (682); intermediate portion (683) may be configured to then receive sideport (530) and to be guided therealong until sideport (530) reaches a proximal end of intermediate portion (683); and locking portion (684) may be configured to then receive and sufficiently capture sideport (530) to inhibit longitudinal movement of cap (670) and sheath hub (500) relative to each other (e.g., to inhibit proximal translation of cap (670) relative to sheath hub (500)) in the absence of an accompanying rotational force applied between cap (670) and sheath hub (500). The bayonet-style engagement between sideport (530) and slot (680) may facilitate secure coupling of cap (670) to sheath hub (500), with a longitudinal axis of insert member assembly (602) and, more particularly, a longitudinal axis of lumen (660) aligned with the longitudinal axis (LA) of insertion port (514).

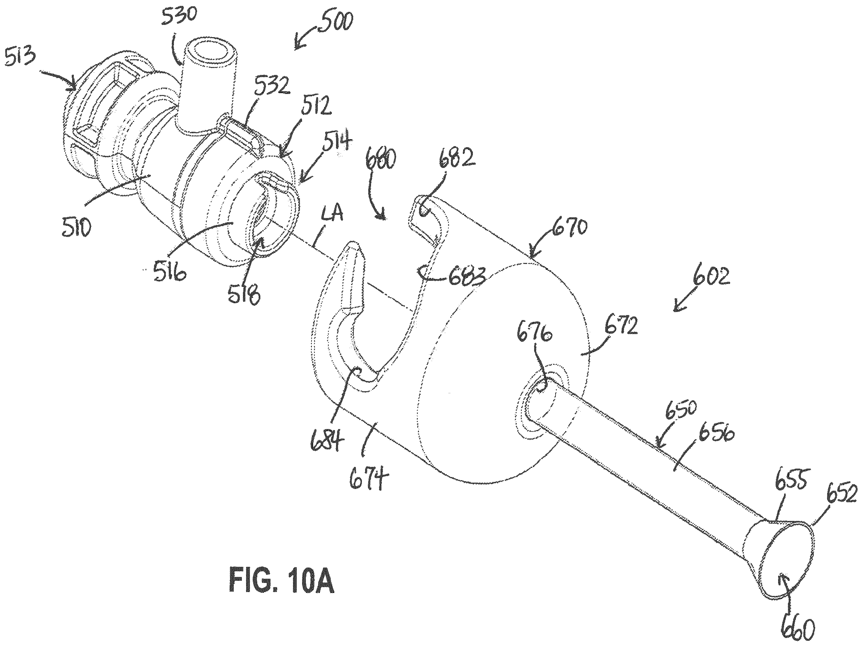

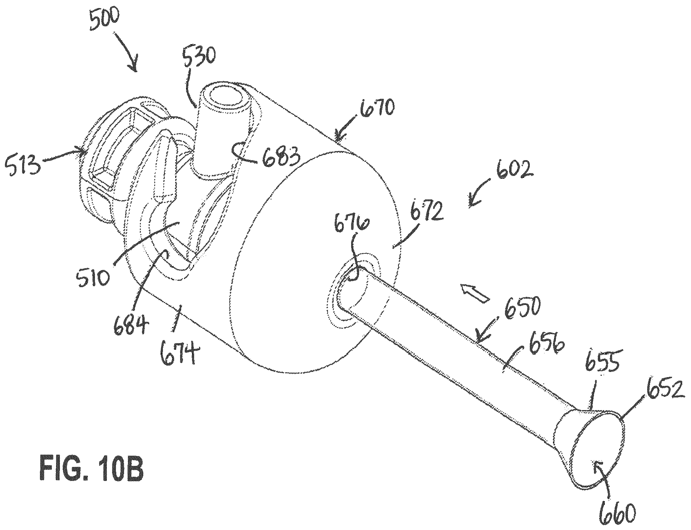

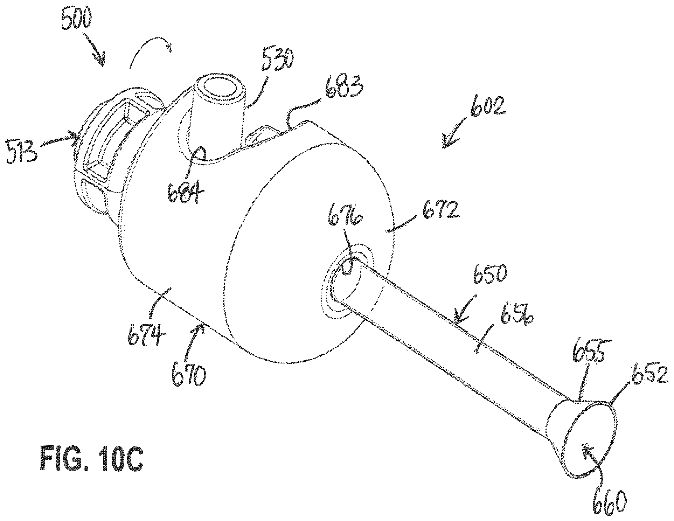

In an example of use of insert member assembly (602), the longitudinal axis (LA) of insert member assembly (602) may be aligned with the longitudinal axis (LA) of insertion port (514), and entryway portion (682) of slot (680) may be aligned with sideport (530), as shown in FIG. 10A. Next, the physician (PH) may advance insert member assembly (602) distally toward insertion port (514), such that body (510) of sheath hub (500) is at least partially received between the generally cylindrical inner surface of sidewall (674) of cap (670) and the generally cylindrical outer surface of main portion (656) of tube (650), and such that sideport (530) of sheath hub (500) is at least partially received within entryway portion (682) of slot (680), as shown in FIG. 10B. The physician (PH) may then rotate cap (670) relative to sheath hub (500) to engage slot (680) of cap (670) with sideport (530) of sheath hub (500) in a bayonet-style manner by twisting intermediate portion (683) of slot (680) along sideport (530) and capturing sideport (530) within locking portion (684) of slot (680) and thereby secure cap (670) to sheath hub (500), as shown in FIG. 10C.

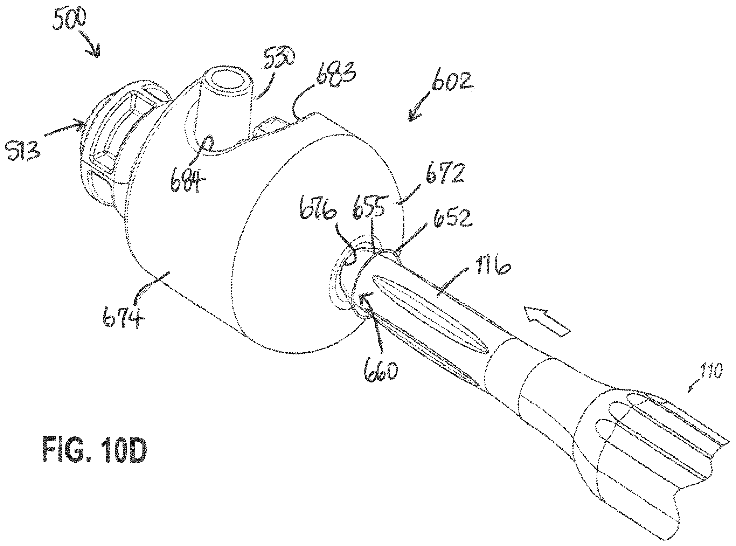

Once cap (670) has been secured to sheath hub (500), catheter (120) may be slid through lumen (660) of insert member assembly (602), and catheter (120) and/or tube (650) may be advanced distally such that end effector (140) and/or distal end (654) penetrate seal (520) at slit arrangement (522) in a manner similar to that described above in connection with FIGS. 4A-4C. In the event that the physician (PH) continues to advance catheter assembly (100) distally to the point where nozzle member (116) of handle assembly (110) engages insert member assembly (602), flared proximal portion (655) of tube (650) may eventually engage end wall (672) of cap (670), as shown in FIG. 10D. For example, the interaction between flared proximal portion (655) of tube (650) and the periphery of central bore (676) will arrest tube (650) and thereby prevent tube (650) from advancing further distally into insertion port (514).

While tube (650) is shown in FIGS. 10C-10D as being slidable relative to cap (670), some variations may provide a fixed relationship between tube (650) and cap (670), such that tube (650) need not necessarily be slidable relative to cap (670) in all versions. For instance, tube (650) and cap (670) may be integrally formed together with each other as a unitary (e.g., monolithic) piece to define insert member assembly (602). In this regard, insert member assembly (602) may be manufactured via 3D printing, injection molding, investment casting, machining, and/or any other suitable manufacturing techniques. In other versions, tube (650) and cap (670) are individually formed separately from each other as discrete parts and coupled to each other to form insert member assembly (602). Alternatively, tube (650) and cap (670) may have any other suitable relationships with each other and may be formed and/or coupled together using any other suitable techniques.

### D. Example of Distal Cap Having Slanted Bayonet Slot for Insert Member Assembly

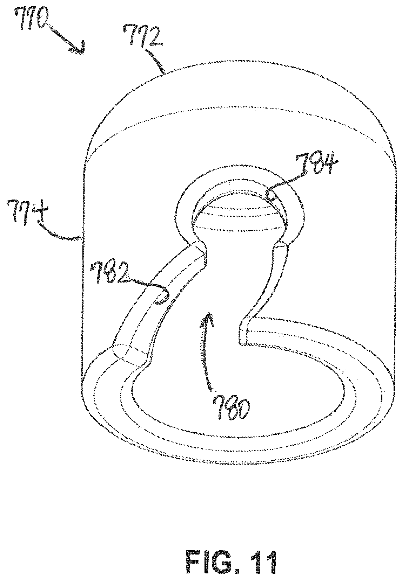

FIG. 11 shows another example of a coupling member in the form of a distal cap (770) that may be incorporated into an insert member assembly like insert member assembly (602) in place of cap (670), and that may be selectively secured to sheath hub (500) and used in a manner similar to that described above for cap (670), such that an insert member assembly incorporating cap (770) may be selectively coupled with delivery sheath (200) via sheath hub (500). Cap (770) is similar to cap (670) described above, except as otherwise described below. In this regard, cap (770) of this example includes a generally dome-shaped proximal end wall (772) and a generally cylindrical distal sidewall (774). While not shown, cap (770) may also include a central bore extending longitudinally through end wall (772) and configured to slidably receive a portion of a tube, such as main portion (656) of tube (650), to facilitate longitudinal sliding of tube (650) relative to cap (770). Alternatively, a tube like tube (650) may be rigidly secured relative to cap (770).

Cap (770) of the present example further includes a bayonet feature in the form of a generally slanted keyhole-shaped slot (780) extending radially outwardly from a generally cylindrical inner surface of sidewall (774) to a generally cylindrical outer surface of sidewall (774) for engaging sideport (530) of sheath hub (500) in a bayonet-style manner. While a single slot (780) is shown, other versions may include any other suitable number of slots (780) such as for allowing a particular slot (780) to be selected for engaging sideport (530). In the example shown, slot (780) includes a slanted entryway portion (782) extending proximally and obliquely from a distal end of cap (770) (e.g., in a clockwise direction within the frame of reference of FIG. 11), and a closed-ended locking portion (784) extending proximally from entryway portion (782) for promoting the bayonet-style engagement between slot (780) and sideport (530). For example, entryway portion (782) may be configured to receive sideport (530) and to be guided therealong until sideport (530) reaches a proximal end of entryway portion (782); and locking portion (784) may be configured to then receive and sufficiently capture sideport (530) to inhibit longitudinal movement of cap (770) and sheath hub (500) relative to each other (e.g., to inhibit proximal translation of cap (770) relative to sheath hub (500)) in the absence of an accompanying rotational force applied between cap (770) and sheath hub (500). The bayonet-style engagement between sideport (530) and slot (780) may facilitate secure coupling of cap (770) to sheath hub (500). In some versions, locking portion (782) may be sized and configured to provide a snap-fit engagement with sideport (530) to assist with facilitating the secure coupling of cap (770) to sheath hub (500).

### E. Example of Insert Member Assembly with Distal Cap Having Cylindrical Sidewall with Snap-Fit Slots

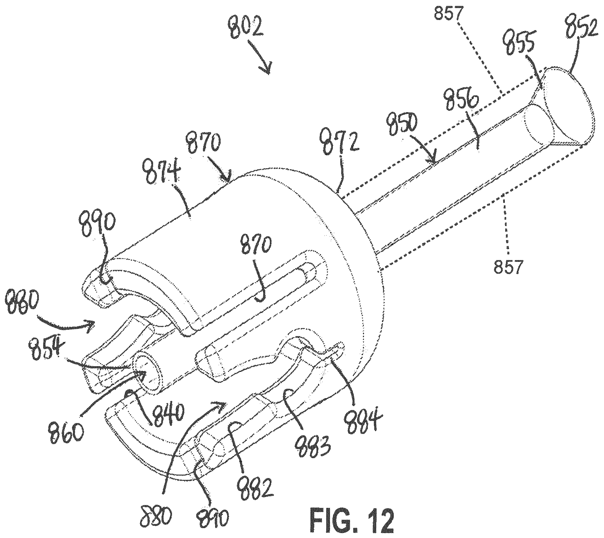

FIGS. 12-13C show another example of an insert member assembly (802) that may be selectively secured to sheath hub (500) and used in a manner similar to that described above for insert member (300), such that insert member assembly (802) may be selectively coupled with delivery sheath (200) via sheath hub (500). Insert member assembly (802) is similar to insert member assembly (502) described above, except as otherwise described below. In this regard, insert member assembly (802) of this example includes a substantially rigid tube (850) extending longitudinally between a proximal end (852) and a distal end (854). In some versions, the rigidity of tube (850) may be enhanced by providing thickened sidewalls, as indicated by broken lines (857) in FIG. 12. Tube (850) has a flared proximal portion (855) and a cylindrical main portion (856), which collectively define a lumen (860) that is sized to closely complement the outer diameter of catheter (120) while permitting catheter (120) to slide freely through tube (850). Lumen (860) may be sized to constrain end effector (140) to the non-expanded state while end effector (140) is positioned within lumen (860). In some variations, proximal end (852) and distal end (854) are both flared. In some other variations, distal end (854) is flared but proximal end (852) is not flared. Proximal end (852) and/or distal end (854) may still provide a conical lead-in/lead-out in versions where tube has thickened sidewalls as indicated by broken lines (857).

Insert member assembly (802) of the present example further includes a coupling member in the form of a distal cap (870) having a generally dome-shaped proximal end wall (872) and a generally cylindrical distal sidewall (874). In the example shown, cap (870) includes a central bore (876) extending longitudinally through end wall (872) and configured to slidably receive main portion (856) of tube (850) to facilitate longitudinal sliding of tube (850) relative to cap (870). In still other variations tube (850) is rigidly secured relative to cap (870), such that tube (850) is not slidable relative to cap (870).

Cap (870) of the present example further includes a pair of laterally-opposed snap-fit features in the form of generally keyhole-shaped slots (880) each extending radially through sidewall (874) for engaging sideport (530) of sheath hub (500) in a snap-fit manner. While a pair of slots (880) is shown such that either slot (880) may be selected for engaging sideport (530), other versions may include only a single slot (880) or any other suitable number of slots (880). In the example shown, each slot (880) includes a distal entryway portion (882) extending proximally from a distal end of cap (870), an intermediate expandable locking portion (883) extending proximally from entryway portion (882), and a proximal closed-ended elongate portion (884) extending proximally from locking portion (883) for promoting the snap-fit engagement between slot (880) and sideport (530). For example, entryway portion (882) may be configured to receive sideport (530) and to be guided therealong until sideport (530) reaches a proximal end of entryway portion (882); locking portion (883) may be configured to then expand sufficiently to receive sideport (530) and resiliently contract sufficiently to capture sideport (530) to inhibit longitudinal movement of cap (870) and sheath hub (500) relative to each other (e.g., to inhibit proximal translation of cap (870) relative to sheath hub (500)) in the absence of a threshold longitudinal force applied between cap (870) and sheath hub (500); and elongate portion (884) may be configured to assist with enabling such expansion of locking portion (883). Entryway portion (882) may be tapered or curved inwardly in a proximal direction to be cammingly engaged by sideport (530) when entryway portion (882) is guided therealong to assist with the expansion of locking portion (883). In the example shown, cap (870) also includes pairs of elongate slots (890) flanking each of the keyhole-shaped slots (880) to enable the portions of sidewall (874) between each of the keyhole-shaped slots (880) and the corresponding elongate slot (890) to deflect into the corresponding elongate slot (890), such as during expansion of the respective locking portion (883).

It will be appreciated that the snap-fit features of sheath hub (500) and cap (870) may be of any suitable form for engaging each other in a snap-fit manner and are not limited to the particular configurations of sideport (530) and slot (880) shown. For example, while sideport (530) is configured to engage slot (880) in a snap-fit manner in the present version, in some other versions sheath hub (500) may include a separate protrusion extending radially outwardly from body (510) and configured to engage slot (880) in a snap-fit manner. The snap-fit engagement between sideport (530) or any other such protrusion and slot (880) may facilitate secure coupling of cap (870) to sheath hub (500), with a longitudinal axis of insert member assembly (802) and, more particularly, a longitudinal axis of lumen (860) aligned with the longitudinal axis (LA) of insertion port (514). In this manner, lumen (860) may be centered relative to slit arrangement (522) of seal (520) and may be oriented orthogonally relative to a plane defined by seal (520) when cap (870) is secured to sheath hub (500).

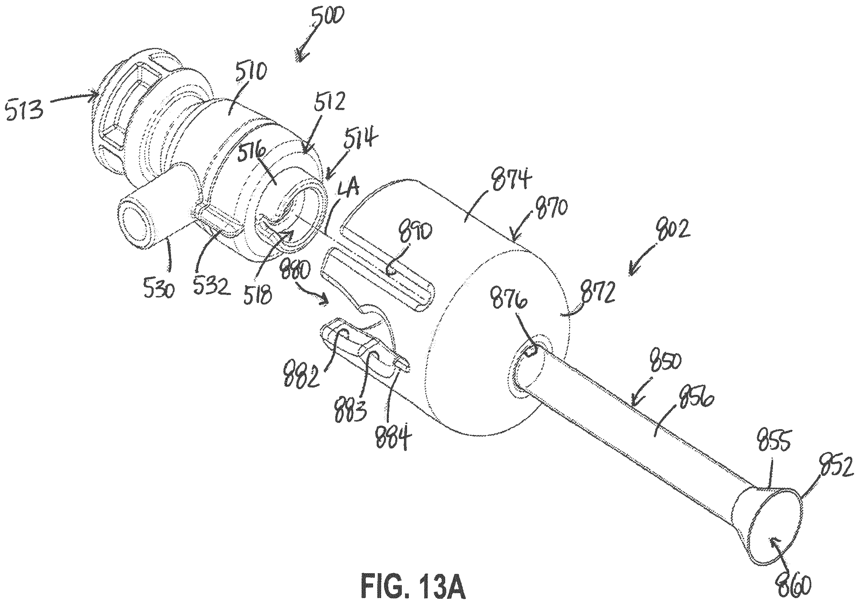

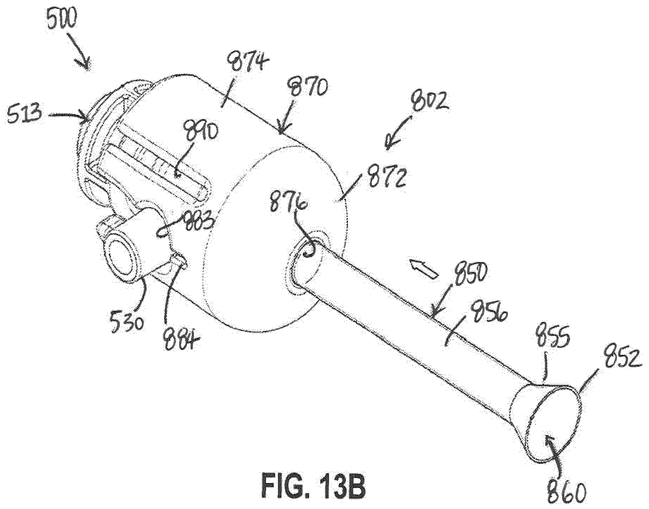

In an example of use of insert member assembly (802), the longitudinal axis (LA) of insert member assembly (802) may be aligned with the longitudinal axis (LA) of insertion port (514), and entry way portion (882) of either slot (880) may be aligned with sideport (530), as shown in FIG. 13A. Next, the physician (PH) may advance insert member assembly (802) distally toward insertion port (514), such that body (510) of sheath hub (500) is at least partially received between the generally cylindrical inner surface of sidewall (874) of cap (870) and the generally cylindrical outer surface of main portion (856) of tube (850), and such that sideport (530) of sheath hub (500) is at least partially received within entryway portion (882) of slot (880), and may continue to advance insert member assembly (802) distally to engage slot (880) of cap (870) with sideport (530) of sheath hub (500) in a snap-fit manner by capturing sideport (530) within locking portion (883) of slot (880) and thereby secure cap (870) to sheath hub (500), as shown in FIG. 13B.

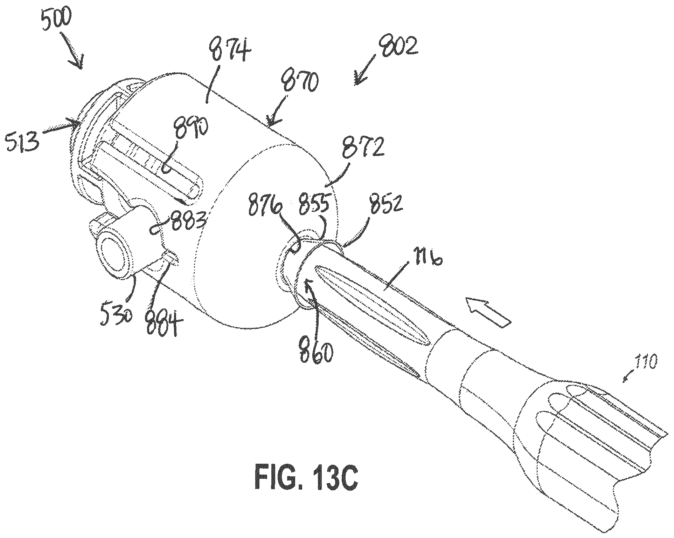

Once cap (870) has been secured to sheath hub (500), catheter (120) may be slid through lumen (860) of insert member assembly (802), and catheter (120) and/or tube (850) may be advanced distally such that end effector (140) and/or distal end (854) penetrate seal (520) at slit arrangement (522) in a manner similar to that described above in connection with FIGS. 4A-4C. In the event that the physician (PH) continues to advance catheter assembly (100) distally to the point where nozzle member (116) of handle assembly (110) engages insert member assembly (802), flared proximal portion (855) of tube (850) may eventually engage end wall (872) of cap (870), as shown in FIG. 13C. For example, the interaction between flared proximal portion (855) of tube (850) and the periphery of central bore (876) will arrest tube (850) and thereby prevent tube (850) from advancing further distally into insertion port (514).

While tube (850) is shown in FIGS. 13B-13C as being slidable relative to cap (870), some variations may provide a fixed relationship between tube (850) and cap (870), such that tube (850) need not necessarily be slidable relative to cap (870) in all versions. For instance, tube (850) and cap (870) may be integrally formed together with each other as a unitary (e.g., monolithic) piece to define insert member assembly (802). In this regard, insert member assembly (802) may be manufactured via 3D printing, injection molding, investment casting, machining, and/or any other suitable manufacturing techniques. In other versions, tube (850) and cap (870) are individually formed separately from each other as discrete parts and coupled to each other to form insert member assembly (802). Alternatively, tube (850) and cap (870) may have any other suitable relationships with each other and may be formed and/or coupled together using any other suitable techniques.

### F. Example of Insert Member Assembly with Distal Cap Having Laterally-Opposed Sidewalls with Snap-Fit Slots

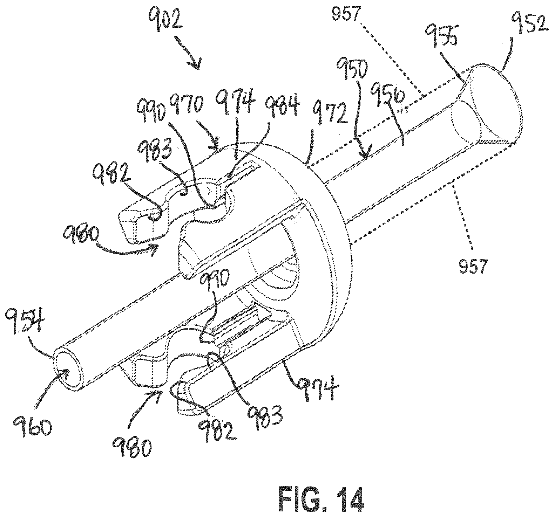

FIG. 14 shows another example of an insert member assembly (902) that may be selectively secured to sheath hub (500) and used in a manner similar to that described above for insert member (300), such that insert member assembly (802) may be selectively coupled with delivery sheath (200) via sheath hub (500). Insert member assembly (902) is similar to insert member assembly (802) described above, except as otherwise described below. In this regard, insert member assembly (902) of this example includes a substantially rigid tube (950) extending longitudinally between a proximal end (952) and a distal end (954). In some versions, the rigidity of tube (950) may be enhanced by providing thickened sidewalls, as indicated by broken lines (957) in FIG. 14. Tube (950) has a flared proximal portion (955) and a cylindrical main portion (956), which collectively define a lumen (960) that is sized to closely complement the outer diameter of catheter (120) while permitting catheter (120) to slide freely through tube (950). Lumen (960) may be sized to constrain end effector (140) to the non-expanded state while end effector (140) is positioned within lumen (960). In some variations, proximal end (952) and distal end (954) are both flared. In some other variations, distal end (954) is flared but proximal end (952) is not flared. Proximal end (952) and/or distal end (954) may still provide a conical lead-in/lead-out in versions where tube has thickened sidewalls as indicated by broken lines (957).

Insert member assembly (902) of the present example further includes a coupling member in the form of a distal cap (970) having a generally dome-shaped proximal end wall (972) and a pair of laterally-opposed, generally arch-shaped distal sidewalls (974). While not shown, cap (970) may also include a central bore (976) extending longitudinally through end wall (972) and configured to slidably receive main portion (956) of tube (950) to facilitate longitudinal sliding of tube (950) relative to cap (970). In still other variations tube (950) is rigidly secured relative to cap (970), such that tube (950) is not slidable relative to cap (970).

Cap (970) of the present example further includes a pair of laterally-opposed snap-fit features in the form of generally keyhole-shaped slots (980) each extending radially through a corresponding sidewall (974) for engaging sideport (530) of sheath hub (500) in a snap-fit manner. While a pair of slots (980) is shown such that either slot (980) may be selected for engaging sideport (530), other versions may include only a single slot (980) or any other suitable number of slots (980). In the example shown, each slot (980) includes a distal entryway portion (982) extending proximally from a distal end of cap (970), an intermediate expandable locking portion (983) extending proximally from entryway portion (982), and a proximal closed-ended elongate portion (984) extending proximally from locking portion (983) for promoting the snap-fit engagement between slot (980) and sideport (530). For example, entryway portion (982) may be configured to receive sideport (530) and to be guided therealong until sideport (530) reaches a proximal end of entryway portion (982); locking portion (983) may be configured to then expand sufficiently to receive sideport (530) and resiliently contract sufficiently to capture sideport (530) to inhibit longitudinal movement of cap (970) and sheath hub (500) relative to each other (e.g., to inhibit proximal translation of cap (970) relative to sheath hub (500)) in the absence of a threshold longitudinal force applied between cap (970) and sheath hub (500); and elongate portion (984) may be configured to assist with enabling such expansion of locking portion (983). Entryway portion (982) may be tapered or curved inwardly in a proximal direction to be cammingly engaged by sideport (530) when entryway portion (982) is guided therealong to assist with the expansion of locking portion (983). In the example shown, cap (970) also includes a pair of laterally-opposed elongate channels (990) each extending along elongate portion (984) of the corresponding slot (980) for slidably receiving tab (532) of sheath hub (500) when sideport (530) is captured within locking portion (983) of the corresponding slot (980). The snap-fit engagement between sideport (530) and slot (980) may facilitate secure coupling of cap (970) to sheath hub (500), with a longitudinal axis of insert member assembly (902) and, more particularly, a longitudinal axis of lumen (960) aligned with the longitudinal axis (LA) of insertion port (514).

### VI. Example of End Effector with Expandable Basket Configuration

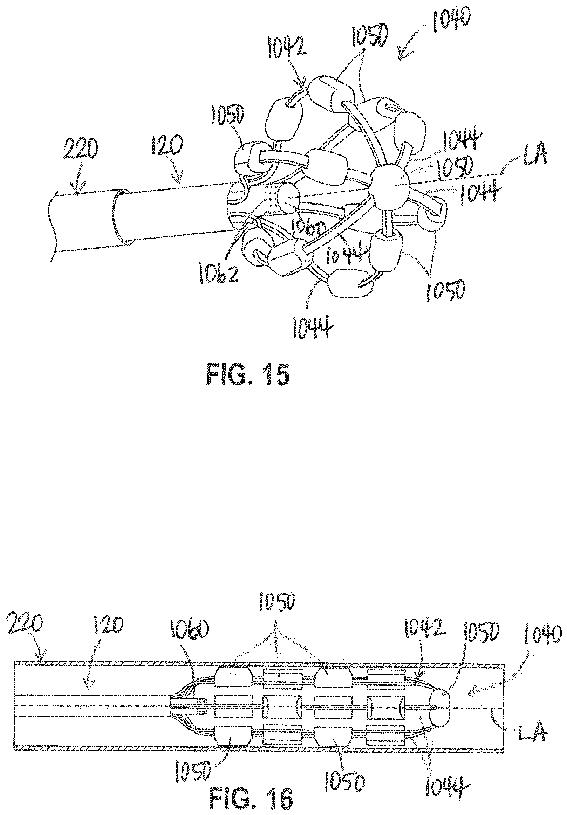

As mentioned above, end effector (140) may have any suitable configuration different from that shown in FIG. 2, such as any suitable type of expandable basket configuration. FIGS. 15-16 show another example of an end effector (1040) having such an expandable basket configuration and which may be incorporated into catheter (120) in place of end effector (140). End effector (1040) is similar to end effector (140) described above, except as otherwise described below. In this regard, end effector (1040) extends distally from an elongate flexible shaft, such as the shaft of catheter (120), and may be selectively positioned within a hollow shaft, such as the hollow shaft (220) of delivery sheath (200).

As shown in FIGS. 15-16, end effector (1040) of the present example includes an expandable basket assembly (1042) having a plurality of resilient spines (1044), and a plurality of electrodes (1050) fitted to spines (1044). Basket assembly (1042) is operable to transition between a non-expanded state (FIG. 16) and an expanded state (FIG. 15). Basket assembly (1042) may be configured to resiliently assume the expanded state when unconstrained, such as by being advanced out of a sheath, such as delivery sheath (200) described above or a sheath that is an integral part of catheter (120). In the non-expanded state, basket assembly (1042) may fit within delivery sheath (200) as shown in FIG. 16. In the expanded state, basket assembly (1042) may be sized and configured to urge electrodes (1050) into contact with tissue (e.g., the inner wall of a pulmonary vein or chamber of the heart (H)).

In the example shown, end effector (1040) also includes a stem (1060) that extends longitudinally from a distal end of the elongate flexible shaft of catheter (120). Stem (1060) of the present example includes a plurality of irrigation ports (1062) configured to deliver irrigation fluid to respective electrodes (1050) and/or to tissue contacted by electrodes (1050) (e.g., the inner wall of a pulmonary vein or chamber of the heart (H)).

In addition to the foregoing, end effector (1040) and other aspects of catheter assembly (100) may be configured and operable in accordance with at least some of the teachings of U.S. Pat. App. No. 17/470,751, the disclosure of which is incorporated by reference herein. Alternatively, end effector (1040) may have any other suitable components, features, and capabilities.

### VII. Example of Insert Device for Use with Expandable Basket End Effector

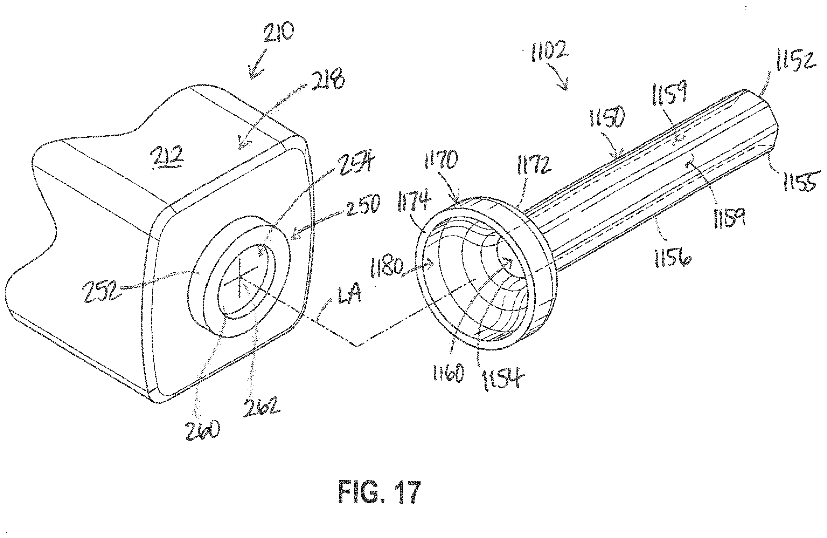

As mentioned above, it may be desirable to enhance the rigidity of the tube of an insert member, such as by providing thickened sidewalls as indicated by broken lines (557, 657, 857, 957) in FIGS. 7, 9, 12, and 14. In addition, or alternatively, it may be desirable to provide an insert member that may be used to assist in inserting end effector (1040) and catheter (120) through insertion port (250) of delivery sheath (200). FIGS. 17-18C show another example of an insert member (1102) that may be selectively secured to handle assembly (210) and used in a manner similar to that described above for insert member (300). Insert member (1102) is similar to insert member (402) described above, except as otherwise described below.

Insert member (1102) of this example includes a tube (1150) extending longitudinally between a proximal end (1152) and a distal end (1154). Tube (1150) has a generally cylindrical proximal portion (1155) and a generally cylindrical main portion (1156). Main portion (1156) has a cylindrical interior while proximal portion (1155) has a frustoconical interior such that the interior of proximal portion (1155) tapers radially outwardly from the interior of main portion (1156) to proximal end (1152) of tube (1150). Main portion (1156) and proximal portion (1155) collectively define a lumen (1160) that extends longitudinally between proximal and distal ends (1152, 1154) of tube (1150) and that is sized to closely complement the outer diameter of catheter (120) while permitting catheter (120) to slide freely through tube (1150). Lumen (1160) may be sized to constrain end effector (1040) to the non-expanded state while end effector (1040) is positioned within lumen (1160). In the present example, tube (1150) is substantially rigid. In this regard, the rigidity of tube (1150) may be enhanced by having thickened sidewalls, at least by comparison to alternative scenarios in which the thickness of the sidewalls of proximal portion (1155) and main portion (1156) are reduced (e.g., so that proximal portion (1155) may be flared in a manner similar to that described above). In the example shown, the sidewalls of tube (1150) include a plurality of flat outer surfaces (1159) configured to be gripped by the physician (PH) or other operator for assisting the operator with manipulating tube (1150) relative to handle assembly (210) and/or catheter assembly (100).

Insert member (1102) of the present example further includes a coupling member in the form of a distal cap (1170) arranged coaxially with tube (1150). Cap (1170) includes a generally flat and/or dome-shaped proximal end wall (1172) extending radially outwardly relative to main portion (1156) of tube (1150) at or near distal end (1154) of tube (1150), and a generally annular distal sidewall (1174).

Cap (1170) of the present example further includes a receptacle (1180) defined by the generally cylindrical inner surface of sidewall (1174) for receiving annular protrusion (252) of insertion port (250). Receptacle (1180) may have a cross dimension (e.g., diameter) slightly less than, substantially equal to, or substantially greater than that of protrusion (252) for allowing protrusion (252) to be received by receptacle (1180). In some versions, the cross dimension of receptacle (1180) may be sized relative to that of receptacle (1180) to allow the generally cylindrical inner surface of sidewall (1174) to frictionally engage the generally cylindrical outer surface of protrusion (252). For example, receptacle (1180) may have a cross dimension slightly less than or substantially equal to that of protrusion (252) for providing an interference fit between the inner surface of sidewall (1174) and the outer surface of protrusion (252).

The frictional engagement between the inner surface of sidewall (1174) and the outer surface of protrusion (252) may facilitate secure coupling of insert member (1102) to handle assembly (210), with a longitudinal axis of insert member (1102) and, more particularly, a longitudinal axis of lumen (1160) aligned with the longitudinal axis (LA) of insertion port (250). In this manner, lumen (1160) may be centered relative to slit arrangement (262) of seal (260) and may be oriented orthogonally relative to a plane defined by seal (260) when insert member (1102) is secured to handle assembly (210). In some versions, the frustoconical interior of proximal portion (1155) of tube (1150) may provide a lead-in for assisting with insertion of end effector (1040) and catheter (120) into proximal end (1152) of tube (1150). In this regard, the frustoconical interior of proximal portion (1155) may have a substantially gradual (e.g., shallow) taper to inhibit kinking of end effector (1040) and catheter (120) during insertion of end effector (1040) and catheter (120). While not shown, cap (1170) may also include at least one vent or bleeder hole extending through end wall (1172) for venting fluid (e.g., air) from a space between a distal surface of end wall (1172) of cap (1170) and handle assembly (210) to an exterior of cap (1170) when cap (1170) is secured to handle assembly (210).

While insert member (1102) has been described as being securely coupled to handle assembly (210) via frictional engagement between the inner surface of sidewall (1174) and the outer surface of protrusion (252), it will be appreciated that insert member (1102) may be securely coupled to handle assembly (210) in any other suitable manner. For example, cap (1170) and/or handle assembly (210) may be equipped with suitable features configured to allow cap (1170) to engage handle assembly (210) in at least one of a threadable manner, a snap-fit manner, an interference-fit manner, or a bayonet-style manner, such as in any of the manners described above. In some other versions, receptacle (1180) may be configured to receive protrusion (252) without insert member (1102) necessarily being securely coupled to handle assembly (210). For example, receptacle (1180) may have a cross dimension substantially greater than that of protrusion (252) for allowing protrusion (252) to loosely fit within receptacle (1180).

Insert member (1102) may be constructed of any suitable translucent (e.g., transparent) or opaque material, such as a polymeric material (e.g., plastic) or a metallic material. For example, insert member (1102) may be constructed of a thermoplastic elastomer, such as polyether block amide (PEBA). In addition, or alternatively, insert member (1102) may be constructed of a material having a Shore durometer of about 63D. Other suitable materials that may be used to form insert member (1102) will be apparent to those skilled in the art in view of the teachings herein. In the example shown, tube (1150) and cap (1170) are integrally formed together with each other as a unitary (e.g., monolithic) piece to define insert member (1102). In this regard, insert member (1102) may be manufactured via 3D printing, injection molding, investment casting, machining, and/or any other suitable manufacturing techniques. In other versions, tube (1150) and cap (1170) are individually formed separately from each other as discrete parts and coupled to each other to define insert member (1102), such that insert member (1102) may be referred to as an assembly. It will be appreciated that tube (1150) and cap (1170) may be constructed of the same or different materials. While tube (1150) of the present example is fixed against movement relative to cap (1170), tube (1150) may alternatively be movable relative to cap (1170). For example, tube (1150) may be slidably coupled to cap (1170) to facilitate longitudinal sliding of tube (1150) relative to cap (1170), such as in a manner similar to any of those described above in connection with FIGS. 7-14.

While not shown, insert member (1102) may include any one or more of a seal member, enhanced structural support features such as ribs, torque-driving features such as external fins, and/or catheter-gripping features such as internal fins. By way of example only, insert member (1102) may be configured and operable in accordance with at least some of the teachings of U.S. Pub. No. 2021/0113812, entitled "Flared Insert Member for Use with Catheter Assembly," published April 22, 2021, the disclosure of which is incorporated by reference herein, in its entirety.

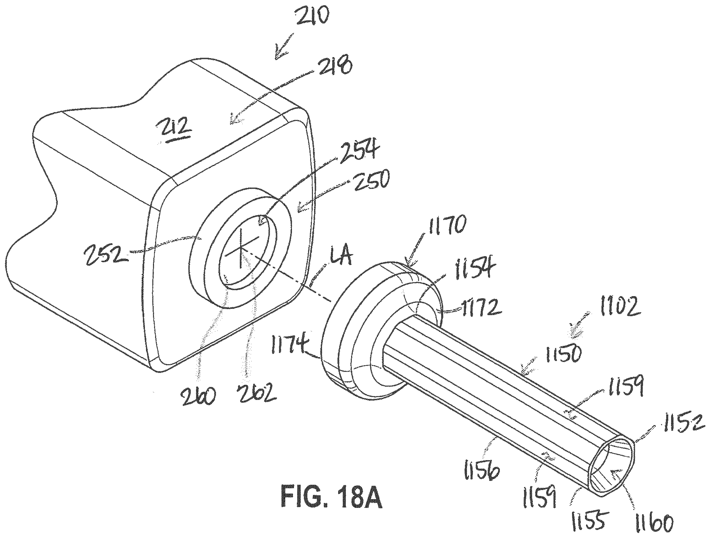

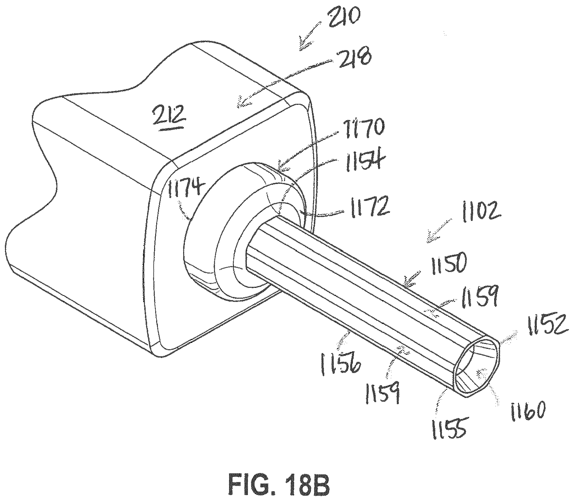

In an example of use of insert member (1102), the longitudinal axis (LA) of insert member (1102) may be aligned with the longitudinal axis (LA) of insertion port (250), as shown in FIG. 18A. Next, the physician (PH) may advance insert member (1102) distally toward insertion port (250), such that annular protrusion (252) of insertion port (250) is at least partially received within receptacle (1180) to frictionally engage the inner surface of sidewall (1174) with the outer surface of protrusion (252) and thereby secure insert member (1102) to handle assembly (210), as shown in FIG. 18B.

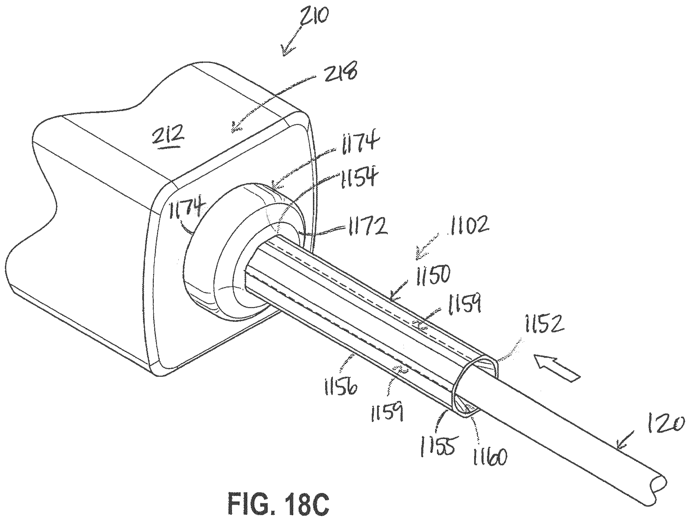

When insert member (1102) is in the secured state shown in FIG. 18B, the longitudinal axis of lumen (1160) is aligned with the longitudinal axis (LA) of insertion port (250), such that lumen (1160) is centered relative to slit arrangement (262) of seal (260) and oriented orthogonally relative to the plane defined by seal (260). It will be appreciated that seal (260) may remain unpenetrated during securing of insert member (1102) to handle assembly (210), such that the securing of insert member (1102) to handle assembly (210) may be performed before inserting end effector (1040) and catheter (120) into insert member (1102). Once insert member (1102) has been secured to handle assembly (210), the longitudinal axis (LA) of end effector (1040) and catheter (120) may be aligned with the longitudinal axis (LA) of insert member (1102) and insertion port (250), and catheter (120) is slid through lumen (1160) of insert member (1102) and advanced distally beyond distal end (1154) to penetrate seal (260) at slit arrangement (262) such that end effector (1040) and catheter (120) may pass distally through the interior of shaft (220), as shown in FIG. 18C. End effector (1040) eventually reaches a point where end effector (1040) is distal to distal end (240) of shaft (220) such that end effector (1040) may be resiliently transitioned to the expanded state. Catheter (120) may be collapsed within shaft (220) as shown in FIG. 16 while insert member (1102) is brought into the secured state show in FIG. 18B.

While insert member (1102) has been described for use with end effector (1040), insert member (1102) may additionally or alternatively be used with any other suitable type of end effector, such as end effector (140) described above.

### VIII. Examples of Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

A delivery sheath to catheter coupler, the coupler comprising: (a) a cylindrical shaft extending along an axis, the cylindrical shaft being configured for longitudinal alignment with an insertion port of a catheter delivery sheath, the cylindrical shaft including: (i) a proximal end, (ii) a distal end, and (iii) a lumen extending from the proximal end to the distal end along the axis, the lumen being sized to receive an end effector of a catheter; and (b) a coupling member attached to the cylindrical shaft, the coupling member being configured for selective securement to the catheter delivery sheath, the coupling member being configured to align the axis of the lumen with a longitudinal axis of the insertion port when the coupling member is selectively secured to the catheter delivery sheath.

### Example 2

The coupler of Example 1, the coupling member being fixedly attached to the cylindrical shaft.

### Example 3

The coupler of Example 1, the coupling member being attached to the cylindrical shaft while also being movable relative to the cylindrical shaft.

### Example 4

The coupler of Example 3, the coupling member being configured to translate relative to the cylindrical shaft along the axis of the lumen.

### Example 5

The coupler of any of Examples 1 through 4, the cylindrical shaft being sized for insertion into the insertion port of the catheter delivery sheath.

### Example 6

The coupler of any of Examples 1 through 5, further comprising an outwardly flared feature at one of the proximal or distal ends of the cylindrical shaft, the outwardly flared feature defining an angled surface leading into the lumen.

### Example 7

The coupler of Example 6, the outwardly flared feature being at the distal end of the cylindrical shaft, the outwardly flared feature being configured to prevent insertion of the cylindrical shaft into the insertion port of the catheter delivery sheath.

### Example 8

The coupler of Example 6, the outwardly flared feature being at the proximal end of the cylindrical shaft, the outwardly flared feature being configured to limit insertion of the cylindrical shaft into the insertion port of the catheter delivery sheath.

### Example 9

The coupler of any of Examples 1 through 8, the coupling member including a vent opening for venting fluid from a space between the coupling member and the catheter delivery sheath when the coupling member is selectively secured to the catheter delivery sheath.

### Example 10

The coupler of any of Examples 1 through 9, the coupling member including a cap.

### Example 11

The coupler of any of Examples 1 through 10, the coupling member being configured to engage the catheter delivery sheath in a threadable manner.

### Example 12

The coupler of any of Examples 1 through 10, the coupling member being configured to engage the catheter delivery sheath in a snap-fit manner.

### Example 13

The coupler of any of Examples 1 through 10, the coupling member being configured to engage the catheter delivery sheath in a bayonet-style manner.

### Example 14

The coupler of any of Examples 1 through 13, further comprising a catheter disposed in the lumen, the catheter being configured to fit within an anatomical structure.

### Example 15

The coupler of any of Examples 1 through 14, further comprising a catheter delivery sheath having an insertion port configured for longitudinal alignment with the cylindrical shaft, the insertion port defining a longitudinal axis, the coupling member being configured to align the axis of the lumen with the longitudinal axis of the insertion port when the coupling member is selectively secured to the catheter delivery sheath.

### Example 16

A kit, comprising: (a) a catheter instrument including: (i) a catheter having a distal end, and (ii) an end effector at the distal end of the catheter, the catheter and the end effector being sized to fit in an anatomical structure, the end effector including at least one electrode; and (b) an coupler including: (i) a cylindrical shaft including: (A) a proximal end, (B) a distal end, and (C) a lumen extending from the proximal end to the distal end along an axis, the lumen being sized to receive the end effector and the catheter, and (ii) a coupling member attached to the cylindrical shaft.

### Example 17

The kit of Example 16, further comprising a catheter delivery sheath, the catheter delivery sheath including an insertion port defining a longitudinal axis, the coupling member being configured for selective securement to the catheter delivery sheath, the coupling member being configured to align the axis of the lumen with the longitudinal axis of the insertion port when the coupling member is selectively secured to the catheter delivery sheath.

### Example 18

The kit of Example 17, the coupling member being configured to engage the catheter delivery sheath in at least one of a threadable manner, a snap-fit manner, or a bayonet-style manner.

### Example 19

A kit, comprising: (a) a catheter instrument including: (i) a catheter having a distal end, and (ii) an end effector at the distal end of the catheter, the catheter and the end effector being sized to fit in an anatomical structure, the end effector including at least one electrode; and (b) an coupler including: (i) a cylindrical shaft including: (A) a proximal end, (B) a distal end, and (C) a lumen extending from the proximal end to the distal end along an axis, the lumen being sized to receive the end effector and the catheter, and (ii) a means for coupling attached to the cylindrical shaft.

### Example 20

The kit of Example 19, the means for coupling including threading, a snap fit feature, a bayonet fit feature, or functional equivalents of threading, a snap fit feature, or a bayonet fit feature

### Example 21

A method comprising: securing an coupler to a catheter delivery sheath, the coupler including: (i) a cylindrical shaft including: (A) a proximal end, (B) a distal end, and (C) a lumen extending from the proximal end to the distal end along an axis, the lumen being sized to receive an end effector and catheter of a catheter instrument, and (ii) a coupling member attached to the cylindrical shaft, the act of securing including aligning the axis of the lumen with a longitudinal axis of an insertion port of the catheter delivery sheath.

### Example 22

The method of Example 21, the act of securing including providing at least one of a threadable engagement, a snap-fit engagement, or a bayonet-style engagement between the coupling member and the catheter delivery sheath.

### Example 23

The coupler of any of Examples 1 through 15, the lumen including a frustoconical proximal portion.

### Example 24

The coupler of Example 23, the frustoconical proximal portion extending distally from the proximal end.

### Example 25

The coupler of any of Examples 23 through 24, the frustoconical proximal portion tapering radially inwardly in a distal direction.

### Example 26

The kit of any of Examples 16 through 20, the lumen including a frustoconical proximal portion.

### Example 27

The kit of Example 26, the frustoconical proximal portion extending distally from the proximal end.

### Example 28

The kit of any of Examples 26 through 27, the frustoconical proximal portion tapering radially inwardly in a distal direction.

### Example 29

A delivery sheath to catheter coupler, the coupler comprising: (a) a cylindrical shaft extending along an axis, the cylindrical shaft being configured for longitudinal alignment with an insertion port of a catheter delivery sheath, the cylindrical shaft including: (i) a proximal end, (ii) a distal end, and (iii) a lumen extending from the proximal end to the distal end along the axis, the lumen being sized to receive an end effector of a catheter, the lumen including a frustoconical proximal portion; and (b) a cap attached to the distal end of the cylindrical shaft, the cap being configured for selective alignment with the catheter delivery sheath, the cap being configured to align the axis of the lumen with a longitudinal axis of the insertion port when the coupling member is selectively aligned with the catheter delivery sheath.

### Example 30

The coupler of Example 29, the cap being configured for selective securement to the catheter delivery sheath.

### Example 31

The coupler of any of Examples 29 through 30, the cap being fixedly attached to the distal end of the cylindrical shaft.

### Example 32

The coupler of any of Examples 29 through 30, the cap being attached to the distal end of the cylindrical shaft while also being movable relative to the cylindrical shaft.

### Example 33

The coupler of Example 32, the cap being configured to translate relative to the cylindrical shaft along the axis of the lumen.

### Example 34

The coupler of any of Examples 30 through 33, the cap being configured to engage the catheter delivery sheath in at least one of a threadable manner, a snap-fit manner, an interference-fit manner, or a bayonet-style manner.

### Example 35

The coupler of any of Examples 29 through 34, further comprising a catheter disposed in the lumen, the catheter being configured to fit within an anatomical structure.

### Example 36

The coupler of any of Examples 29 through 35, further comprising a catheter delivery sheath having an insertion port configured for longitudinal alignment with the cylindrical shaft, the insertion port defining a longitudinal axis, the coupling member being configured to align the axis of the lumen with the longitudinal axis of the insertion port when the coupling member is aligned with the catheter delivery sheath.

### IX. Miscellaneous

Any of the instruments described herein may be cleaned and sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, hydrogen peroxide, peracetic acid, and vapor phase sterilization, either with or without a gas plasma, or steam.

It should be understood that any of the examples described herein may include various other features in addition to or in lieu of those described above. By way of example only, any of the examples described herein may also include one or more of the various features disclosed in any of the various references that are incorporated by reference herein.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Having shown and described various versions of the present invention, further adaptations of the methods and systems described herein may be accomplished by appropriate modifications by one skilled in the art without departing from the scope of the present invention. Several of such potential modifications have been mentioned, and others will be apparent to those skilled in the art. For instance, the examples, versions, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A delivery sheath to catheter coupler, the coupler comprising:
(a) a cylindrical shaft extending along an axis, the cylindrical shaft being configured for longitudinal alignment with an insertion port of a catheter delivery sheath, the cylindrical shaft including:
(i) a proximal end,
(ii) a distal end, and
(iii) a lumen extending from the proximal end to the distal end along the axis, the lumen being sized to receive an end effector of a catheter; and
(b) a coupling member attached to the cylindrical shaft, the coupling member being configured for selective securement to the catheter delivery sheath, the coupling member being configured to align the axis of the lumen with a longitudinal axis of the insertion port when the coupling member is selectively secured to the catheter delivery sheath.

2. The coupler of claim 1, the coupling member being fixedly attached to the cylindrical shaft, or being attached to the cylindrical shaft while also being movable relative to the cylindrical shaft, optionally the coupling member being configured to translate relative to the cylindrical shaft along the axis of the lumen.

3. The coupler of claim 1, the cylindrical shaft being sized for insertion into the insertion port of the catheter delivery sheath.

4. The coupler of claim 1, further comprising an outwardly flared feature at one of the proximal or distal ends of the cylindrical shaft, the outwardly flared feature defining an angled surface leading into the lumen.

5. The coupler of claim 4, the outwardly flared feature being at the distal end of the cylindrical shaft, the outwardly flared feature being configured to prevent insertion of the cylindrical shaft into the insertion port of the catheter delivery sheath, or being at the proximal end of the cylindrical shaft, the outwardly flared feature being configured to limit insertion of the cylindrical shaft into the insertion port of the catheter delivery sheath.

6. The coupler of claim 1, the coupling member including a vent opening for venting fluid from a space between the coupling member and the catheter delivery sheath when the coupling member is selectively secured to the catheter delivery sheath, or including a cap.

7. The coupler of claim 1, the coupling member being configured to engage the catheter delivery sheath in a threadable manner, in a snap-fit manner, or in a bayonet-style manner.

8. The coupler of claim 1, further comprising a catheter delivery sheath having an insertion port configured for longitudinal alignment with the cylindrical shaft, the insertion port defining a longitudinal axis, the coupling member being configured to align the axis of the lumen with the longitudinal axis of the insertion port when the coupling member is selectively secured to the catheter delivery sheath.

9. A kit, comprising:
(a) a catheter instrument including:
(i) a catheter having a distal end, and
(ii) an end effector at the distal end of the catheter, the catheter and the end effector being sized to fit in an anatomical structure, the end effector including at least one electrode; and
(b) a coupler including:
(i) a cylindrical shaft including:
(A) a proximal end,
(B) a distal end, and
(C) a lumen extending from the proximal end to the distal end along an axis, the lumen being sized to receive the end effector and the catheter, and
(ii) a coupling member attached to the cylindrical shaft.

10. The kit of claim 9, further comprising a catheter delivery sheath, the catheter delivery sheath including an insertion port defining a longitudinal axis, the coupling member being configured for selective securement to the catheter delivery sheath, the coupling member being configured to align the axis of the lumen with the longitudinal axis of the insertion port when the coupling member is selectively secured to the catheter delivery sheath.

11. The kit of claim 10, the coupling member being configured to engage the catheter delivery sheath in at least one of a threadable manner, a snap-fit manner, or a bayonet-style manner.

12. A kit, comprising:
(a) a catheter instrument including:
(i) a catheter having a distal end, and
(ii) an end effector at the distal end of the catheter, the catheter and the end effector being sized to fit in an anatomical structure, the end effector including at least one electrode; and
(b) a coupler including:
(i) a cylindrical shaft including:
(A) a proximal end,
(B) a distal end, and
(C) a lumen extending from the proximal end to the distal end along an axis, the lumen being sized to receive the end effector and the catheter, and
(ii) a means for coupling attached to the cylindrical shaft.

13. The kit of claim 12, the means for coupling including threading, a snap fit feature, a bayonet fit feature, or functional equivalents of threading, a snap fit feature, or a bayonet fit feature.

14. A method comprising:
securing a coupler to a catheter delivery sheath, the coupler including:
(i) a cylindrical shaft including:
(A) a proximal end,
(B) a distal end, and
(C) a lumen extending from the proximal end to the distal end along an axis, the lumen being sized to receive an end effector and catheter of a catheter instrument, and
(ii) a coupling member attached to the cylindrical shaft,
the act of securing including aligning the axis of the lumen with a longitudinal axis of an insertion port of the catheter delivery sheath.

15. The method of claim 14, the act of securing including providing at least one of a threadable engagement, a snap-fit engagement, or a bayonet-style engagement between the coupling member and the catheter delivery sheath.

16. The coupler of claim 1 or the kit of claim 9, the lumen including a frustoconical proximal portion, optionally the frustoconical proximal portion extending distally from the proximal end or tapering radially inwardly in a distal direction.

17. A delivery sheath to catheter coupler, the coupler comprising:
(a) a cylindrical shaft extending along an axis, the cylindrical shaft being configured for longitudinal alignment with an insertion port of a catheter delivery sheath, the cylindrical shaft including:
(i) a proximal end,
(ii) a distal end, and
(iii) a lumen extending from the proximal end to the distal end along the axis, the lumen being sized to receive an end effector of a catheter, the lumen including a frustoconical proximal portion; and
(b) a cap attached to the distal end of the cylindrical shaft, the cap being configured for selective alignment with the catheter delivery sheath, the cap being configured to align the axis of the lumen with a longitudinal axis of the insertion port when the coupling member is selectively aligned with the catheter delivery sheath.

18. The coupler of claim 17, the cap being (i) configured for selective securement to the catheter delivery sheath, optionally the cap being configured to engage the catheter delivery sheath in at least one of a threadable manner, a snap-fit manner, an interference-fit manner, or a bayonet-style manner, (ii) fixedly attached to the distal end of the cylindrical shaft, or (iii) attached to the distal end of the cylindrical shaft while also being movable relative to the cylindrical shaft, optionally the cap being configured to translate relative to the cylindrical shaft along the axis of the lumen.

19. The coupler of claim 1 or claim 17, further comprising a catheter disposed in the lumen, the catheter being configured to fit within an anatomical structure.

20. The coupler of claim 17, further comprising a catheter delivery sheath having an insertion port configured for longitudinal alignment with the cylindrical shaft, the insertion port defining a longitudinal axis, the coupling member being configured to align the axis of the lumen with the longitudinal axis of the insertion port when the coupling member is aligned with the catheter delivery sheath.
